## Europäisches Patentamt
## European Patent Office
## Office européen des brevets

(19)

(11) Veröffentlichungsnummer: **0 181 465 B2**

(12)

# NEUE EUROPÄISCHE PATENTSCHRIFT

(45) Veröffentlichungstag der neue Patentschrift:
**23.11.94**

(21) Anmeldenummer: **85111887.7**

(22) Anmeldetag: **20.09.85**

(51) Int. Cl.5: **C07K 15/06**, C07K 3/28, A61K 37/02, A61K 35/44

(54) **Blutgerinnungshemmende Proteine, Verfahren zur Herstellung sowie deren Verwendung.**

(30) Priorität: **21.09.84 NL 8402904**
**04.03.85 NL 8500601**

(43) Veröffentlichungstag der Anmeldung:
**21.05.86 Patentblatt 86/21**

(45) Bekanntmachung des Hinweises auf die Patenterteilung:
**09.08.89 Patentblatt 89/32**

(45) Bekanntmachung des Hinweises auf die Entsheidung über den Einspruch:
**23.11.94 Patentblatt 94/47**

(84) Benannte Vertragsstaaten:
**AT BE CH DE FR GB IT LI LU NL SE**

(56) Entgegenhaltungen:
EP-A- 0 123 307
EP-A- 0 318 703
DE-A- 2 353 318
DE-A- 3 710 430
US-A- 4 347 243

BIOLOGICAL ABSTRACTS, Band 55, 15. Mai 1973, p. 5441, ref.no. 53441; Philadelphia, US, A.A. KEMOKLIDZE et al.: "Coagulant activity of tissue extracts of the vascular walls."

CHEMICAL ABSTRACTS, Band 100, no. 11, 12. März 1984, pp. 356-7, ref.no. 83398t; Columbus, Ohio, US, K. SHIMADA et al.: "Anticoagulant properties of glycosaminoglycans derived from cultured endothelial cells."

Fusao Hirata, J. Biol. Chem., Bd. 256, No. 15 (1981), 7730-7733

Di Rosa et al., Prostaglandins, Bd. 28 (1984), 441-442

M.J. Geisow et al., Biochem. Soc. Transactions, Bd. 15 (1987), 800-802

Europ.J. Obstet.Gynec. Reprod.Biol. Bd. 17 (2/3), Mai 1984, S. 149-154

Blood and Vessel Bd. 14 (4) April 1984, S. 498-500

(73) Patentinhaber: **BOEHRINGER INGELHEIM INTERNATIONAL GmbH**
**Postfach 200**
**D-55216 Ingelheim (DE)**

(72) Erfinder: **Reutelingsperger, Christian P. M., Drs.**
**Looiersgracht 17**
**NL-6211 JK Maastricht (NL)**

EP 0 181 465 B2

**Beschreibung**

Die vorliegende Erfindung beschreibt Proteine, die die Blutgerinnung hemmen, Verfahren zur Herstellung dieser Proteine sowie ihre Verwendung.

Blutgerinnungshemmende Proteine, die in den meisten Säugetieren vorhanden sind, können in drei Gruppen eingeteilt werden. Diese Einteilung beruht auf dem Unterschied der Protein-Wirkungsmechanismen.

1. Proteine, die mit dem Gerinnungsfaktor einen Komplex bilden und dadurch den Gerinnungsfaktor unwirksam machen. Dazu gehören die Proteine :

    a) Antithrombin-III (Thromb. Res. 5, 439-452 (1974))

    b) $\alpha_1$-Protease Inhibitor (Ann. Rev. Biochem. 52, 655-709 (1983))

    c) $\alpha_2$-Makroglobulin (Ann. Rev. Biochem. 52, 655-709 (1983))

    d) $C_1$-Inhibitor (Biochemistry 20, 2738-2743 (1981))

    e) Protease Nexin (J. Biol. Chem. 258, 10439-10444, (1983)).

2. Proteine, die einen Gerinnungsfaktor proteolytisch zerschneiden und dadurch den Gerinnungsfaktor desaktivieren. Als einziges Protein dieser Art ist bisher Protein C beschrieben (J. Biol. Chem. 251, 355-363 (1976)).

3. Proteine, die die negativ geladenen Phospholipide abschirmen und/oder hydrolisieren, so daß die Phospholipid-abhängigen Reaktionen des Blutgerinnungsmechanismus gehemmt werden. Bisher sind nur Phospholipasen, die aus verschiedenen Schlangengiftsorten isoliert wurden, beschrieben worden (Europ. J. Biochem. 112, 25-32 (1980)).

Das stufenweise ablaufende Gerinnungssystem ist in den letzten Jahren intensiv untergucht worden. Es wird als ein sich verstärkendes Mehrstufensystem verschiedener miteinander verbundener, proteolytischer Reaktionen verstanden, bei dem ein Enzym ein Zymogen in die aktive Form umsetzt (vgl. Jackson C.M., Nemerson Y., Ann. Rev. Biochem. 49, 765-811 (1980)). Die Geschwindigkeit dieser Reaktion wird in entscheidender Weise durch die Anwesenheit von Phospholipiden und anderer Kofaktoren wie Faktor $V_a$ und Faktor $VIII_a$ vergrößert. In vivo werden die Prokoagulationsreaktionen durch verschiedene Inhibitionsmechanismen geregelt, die einem explosiv thrombotischen Trauma nach einer geringen Aktivierung der Gerinnungskaskade vorbeugen.

Die Antigerinnungsmechanismen können wie folgt eingeteilt werden (Rosenberg, R.D., Rosenberg, J.S., J. Clin. Invest. 74, 1-6 (1984)) :

1. Der Serin-Protease-Faktor $X_a$ und Thrombin werden infolge ihrer Bindung an Antithrombin III bzw. an den Antithrombin/Heparin-Komplex inaktiviert. Sowohl die Prothrombin-Aktivierung als auch die Bildung von Fibrin kann auf diese Weise gehemmt werden. Neben Antithrombin III gibt es noch verschiedene andere Plasma-Protease-Inhibitoren wie beispielsweise $\alpha_2$-Makroglobulin und Antitrypsin deren Wirkung zeitabhängig ist.

2. Die Entdeckung des Protein C's führte zur Aufdeckung eines weiteren Antigerinnungsmechanismus. Ist das Protein C einmal aktiviert worden, wirkt es durch die selektive Proteolyse der Proteinkofaktoren Faktor $V_a$ und $VIII_a$, durch die die Prothrombinase und das Enzym, das den Faktor X umsetzt, inaktiviert werden, als Antikoagulanz.

3. Plasmin spaltet monomeres Fibrin 1, ein Produkt der Einwirkung von Thrombin auf Fibrinogen, wodurch der Bildung eines unlöslichen Fibrins vorgebeugt wird (Nossel, H.L., Nature, 291, 165-167 (1981)).

Von den obengenannten, in den Gerinnungsprozeß eingreifenden körpereigenen Proteinen ist z.Zt. nur das Anthribombin III in klinischem Einsatz. Als erheblicher Nachteil hat sich jedoch die bei der Anwendung dieses Proteins auftretende Erhöhung der Blutungsneigung herausgestellt.

Alle bisher als Antikoagulantien eingesetzten Mittel, ob körpereigener oder synthetischer Natur machen in irgendeiner Weise die Gerinnungsfaktoren unwirksam un führen dadurch zu Nebenwirkungen, die sich nachteilig auf den Gerinnungsprozeß auswirken können.

Aufgabe der vorliegenden Erfindung war es, ein Mittel bereitzustellen, das blutgerinnungshemmende Eigenschaften aufweist, jedoch ohne die sich nachteilig auf den Gerinnungsprozeß auswirkenden Eigenschaften der bisher bekannten Antikoagulantien.

Überraschenderweise konnten nun körpereigene Proteine isoliert werden, die blutgerinnungshemmende Eigenschaften aufweisen, nicht jedoch die Blutungsgefahr erhöhen. Als weitere überraschende Eigenschaft verlieren diese Proteine bei größeren Blutungen ihre hemmenden Eigenschaften, so daß die üblichen Gerinnungsprozesse ungestört ablaufen können und die Gefahr eines Verblutens nicht besteht.

Gegenstand der vorliegenden Erfindung sind die im folgenden VAC (Vascular Anti-Coagulant) genannten blutgerinnungshemmenden Proteine gemäss Anspruch 1, die die Gerinnungsfaktoren nicht inaktivieren.

EP 0 181 465 B2

Diese Proteine sind in der Lage, die durch ein vaskuläres Prokoagulanz bzw. durch den Faktor $X_a$ induzierte Gerinnung zu hemmen, jedoch nicht die von Thrombin induzierte Gerinnung. Sie hemmen nicht die biologische und amidolytische Aktivität der Faktoren $X_a$ und $II_a$.

Gegenstand der Erfindung sind blutgerinnungshemmende Proteine, die die Gerinnungsfaktoren nicht inaktivieren und :

den modifizierten Prothrombin-Zeit-Versuch und/oder

den modifizierten, aktivierten, partiellen Thromboplastin-Zeit-Versuch und/oder

den nicht-modifizierten Prothrombin-Zeitversuch und/oder

die Prothrombin-Aktivierung durch den Gerinnungsfaktor $X_a$ in Gegenwart von negativ geladenen Phospholipiden und $Ca^{2+}$ und/oder

die intrinsische X-Aktivierung durch den Faktor $IX_a$ in Gegenwart von negativ geladenen Phospholipi den und $Ca^{2+}$ und/oder die Prothrombin-Aktivierung von isolierten, stimulierten Blutplättchen und/oder

die von der Gefäßwand induzierte Gerinnung und/oder

die gerinnungsabhängige Plättchenaggregation hemmen.

Gegenstand der Erfindung sind auch blutgerinnungshemmende Proteine, die die Gerinnungsfaktoren nicht inaktivieren und deren hemmende Wirkung von der Menge an Phospholipiden abhängt. Die erfindungsgemäßen Proteine induzieren die Prothrombin-Aktivierungshemmung durch den Faktor $X_a$. Die Hemmung ist abhängig von der Phospholipidkonzentration und ist bei hohen Phospholipidkonzentrationen kleiner. Die Phospholipide werden von den erfindungsgemäßen Proteinen nicht hydrolisiert.

Gegenstand der Erfindung sind ferner blutgerinnungshemmende Proteine, die die Gerinnungsfaktoren nicht inaktivieren und die über die divalenten Kationen $Ca^{2+}$ und/oder $Mn^{2+}$ an negativ geladene Phospholipide, die beispielsweise in Vesikeln, Liposomen oder Etherosomen zu finden sind, und/oder über die divalenten Kationen $Ca^{2+}$ und $Mn^{2+}$ an negativ geladene Phospholipide, die mit Spherocil gekoppelt sind binden. Die Bindung der Proteine an die negativ geladenen Phospholipide ist reversibel und kann von Ethylendiamintetraessigsäure (EDTA) aufgehoben werden. Die erfindungsgemäßen Proteine sind in der Lage, den Faktor $X_a$ und die Prothrombine von einer negativ geladenen Phospholipidoberfläche zu verdrängen.

Gegenstand der Erfindung sind insbesondere blutgerinnungshemmende Proteine, die die Gerinnungsfaktoren nicht inaktivieren und Molekulargewichte von ca. $70 \times 10^3$, $60 \times 10^3$, $34 \times 10^3$ oder $32 \times 10^3$ aufweisen, von denen die Proteine mit dem Molekulargewicht $34 \times 10^3$ respektive $32 \times 10^3$ aus jeweils nur einer einzigen Polypeptidkette bestehen.

Gegenstand der Erfindung sind vorzugsweise blutgerinnungshemmende Proteine, die die Gerinnungsfaktoren nicht inaktivieren, dadurch gekennzeichnet, daß

sie aus Blutgefäßwänden von Säugetieren isoliert und gereinigt wurden,

sie keine Glykoproteine sind,

sie keine Phospholipasen sind,

sie einen isoelektrischen Punkt bei pH 4,4-4,6 besitzen,

die Aktivität der blutgerinnungshemmenden Proteine bei 56 °C thermolabil ist,

die Aktivität der blutgerinnungshemmenden Proteine im zitrierten Plasma bei 37 °C einige Stunden stabil bleibt,

die Aktivität der blutgerinnungshemmenden Proteine von Trypsin und/oder Chymotrypsin nicht vollständig zerstört wird,

die Aktivität der blutgerinnungshemmenden Proteine von Collagenase und/oder Elastase nicht beeinflußt wird,

sie über die divalenten Kationen $Ca^{2+}$ und $Mn^{2+}$ mit negativ geladenen Phospholipiden, die in Vesikeln, Liposomen oder Etherosomen zu finden sind, binden,

sie über die divalenten Kationen $Ca^{2+}$ und $Mn^{2+}$ mit negativ geladenen Phospholipiden, die mit Spherocil gekoppelt sind, binden,

die Bindung der Proteine an die negativ geladenen Phospholipide reversibel ist und von Ethylendiamintetraessigsäure (EDTA) aufgehoben werden kann,

sie den Faktor $X_a$ und die Prothrombine von einer negativ geladenen Phospholipidoberfläche verdrängen,

sie den modifizierten Thrombin-Zeit-Versuch hemmen,

sie den modifizierten, aktivierten, partiellen Thromboplastin-Zeit-Versuch hemmen,

sie den nicht-modifizierten Prothrombin-Zeitversuch hemmen,

sie die Prothrombin-Aktivierung durch den Gerinnungsfaktor $X_a$ in Gegenwart von negativ geladenen Phospholipiden und $Ca^{2+}$ in vitro hemmen,

sie nicht die biologische und amidolytische Aktivität der Faktoren $X_a$ und $II_a$ hemmen,

3

EP 0 181 465 B2

sie die intrinsische X-Aktivierung durch den Faktor $IX_a$ in Gegenwart von negativ geladenen Phospholipiden und $Ca^{2+}$ in vitro hemmen,

sie die Prothrombin-Aktivierung von isolierten, stimulierten Blutplättchen in vitro hemmen,

sie die von der Gefäßwand induzierte Gerinnung in vitro hemmen und

die durch die Proteine induzierte Prothrombin-Aktivierungshemmung durch den Faktor $X_a$ von der Phospholipidkonzentration abhängig ist und bei hohen Phospholipidkonzentrationen kleiner ist.

Gegenstand der Erfindung sind insbesondere VAC-Proteine, im wesentlichen frei von jedwedem tierischem Gewebe. vorzugsweise in im wesentlichen reiner Form.

Geeignete Ausgangsmaterialien für die Isolierung der VAC-Proteine sind die Blutgefäßwände sowie stark vaskularisiertes Gewebe verschiedener Säugetiere beispielsweise von Rindern, Ratten, Pferden und Menschen sowie Endothelzellkulturen dieser Säugetiere. Insbesondere sind die Arterienwände von Rindern, Ratten, Pferden und Menschen wie auch humane Nabelschnurvenen und -arterien geeignet.

Gegenstand der Erfindung ist auch ein Verfahren zur Herstellung des erfindungsgemäßen Proteins mittels an sich bekannter Isolierungs- und Reinigungstechniken. Insbesondere geeignet ist folgendes Verfahrensschema :

Das homogenisierte Ausgangsmaterial wird zunächst einer Differentialzentrifugation ausgesetzt. Die erhaltene überstehende Flüssigkeit kann dann nacheinander in beliebiger Reihenfolge folgendermaßen weiterbehandelt werden. Mit Ammoniumsulfat können unerwünschte Beimengungen präzipitiert werden. Der Überstand wird dann mittels Affinitätschromatographie, beispielsweise über Hydroxyapatit, Ionenaustauschchromatographie, z. B. über DEAE-Sephacel, Chromatographie über ein Molekularsieb, wie Sephadex G-100 und Immunadsorptionschromatographie, beispielsweise mit polyklonalen oder auch monoklonalen Antikörpern weiter gereinigt. Je nach der Qualität des Ausgangsmaterials kann dieses Reinigungsschema abgewandelt oder auch andere Reinigungsverfahren angewendet werden, beispielsweise die Reinigung mit Hilfe von Phospholipid-Vesikeln.

Neben der klassischen Antithrombosebehandlung, der oralen Antigerinnung, wird neuerdings für den Fall einer manifesten Thrombose biosynthetischer Gewebe-Plasminogenaktivator intravaskulär verabreicht (N. Engl. J. Med. 310, 609-613 (1984)).

Die erfindungsgemäßen Proteine sind hervorragend geeignet, insbesondere durch die blutgerinnungshemmenden Eigenschaften gleichzeitig mit der Hemmung der gerinnungsabhängigen Plättchenaggregation, einer Thrombose, beispielsweise bei Operationen vorzubeugen.

Gegenstand der vorliegenden Erfindung ist daher ebenfalls die Verwendung der erfindungsgemäßen Proteine als antithrombotisches Mittel.

Ein weiterer Gegenstand der Erfindung sind auch pharmazeutische Präparate, die zumindest ein erfindungsgemäßes Protein enthalten.

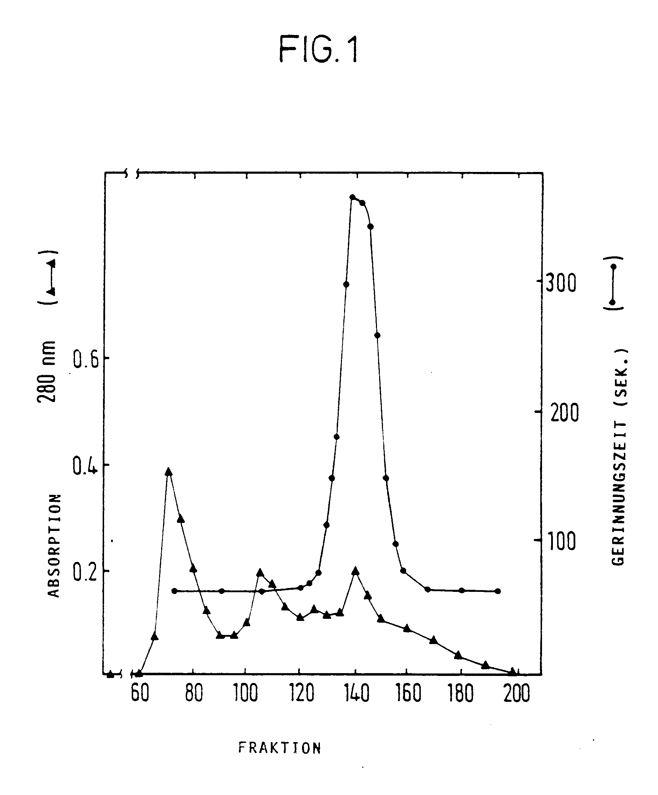

Die Ergebnisse der VAC-Isolierung und -Reinigung aus Rinderaorten sind in der Tabelle A aufgeführt. Die Bestimmung der Menge an VAC-Aktivität im Überstand der 100.000 xg Zentrifugation war wegen der Gegenwart einer Prokoagulanzaktivität irrelevant. Die für diese Aktivität verantwortlichen Bestandteile konnten mit Ammoniumsulfat bei einem Sättigungsgrad von 35 % präzipitiert werden. Festgestellt werden konnte, daß die obenstehende Lösung nach dem Präzipitieren mit 35 % Ammoniumsulfat 100 % VAC-Aktivität enthielt. Zur Konzentrierung wurde die Lösung bis zu einer Sättigung von 90 % mit Ammoniumsulfat versetzt. Das Präzipitat, die VAC-Proteine enthaltend, wurde in Gegenwart von TBS an eine Hydroxyapatit-Säule gebunden. Nach dem Waschen wurden die VAC-Proteine aus dieser Säule mit einem steigenden Phosphatgradienten eluiert. Bei einer niedrigen Ionenstärke wurden die VAC-Proteine an eine DEAE-Sephacel-Säule gebunden. Die Elution der VAC-Proteine von dieser Säule erfolgte mit einem zunehmenden NaCl-Konzentrationsgradienten. Bei der Endreinigung wurden die Proteine auf Grund ihres Molekulargewichtes durch die Gelfiltration über Sephadex G-100 getrennt. Das Eluenz war ein hochsalziger Puffer zum Minimalhalten der Interaktion von VAC mit dem Sephadex-Material. VAC eluierte aus dieser Säule in einem Volumen von etwa 1,6 mal des leeren Säulenvolumens (siehe Figur 1). Die Gesamtausbeute der VAC-Aktivität nach dieser Endreinigung betrug 35 %. Gemäß der SDS-PAGE enthalten alle G-100 Fraktionen, die VAC-Aktivität zeigten, zwei Polypeptide (Molekulargewicht 34.000 bzw. 32.000). In einigen Fällen zeigte auch eine Fraktion mit dem Molekulargewicht 60.000 VAC-Aktivität. Gemäß SDS-PAGE sind nur die Spitzenfraktionen 138-140 homogen in Bezug auf die beiden Polypeptide.

In der Tabelle A wurden die Reinigungsdaten aus den Rinderaorten zusammengefaßt. Die G-100 Fraktionen, die in Bezug auf die beiden Polypeptide homogen sind, wurden für alle weiteren Experimente dieser Beschreibung verwendet, mit Ausnahme der Experimente zur Charakterisierung der Bindung von VAC an Phospholipid-Liposome.

In der G-100 Fraktion 139 wurden 3,4 % der VAC-Aktivität mit einer spezifischen Aktivität von 1 480 Einheiten/mg Protein ermittelt, wie mit dem Einstufenkoagulationsversuch festgestellt werden konnte. Diese

4

Fraktion enthielt keine nachweisbare Menge an Phospholipid und aus der Absorption bei 280 nm und dem Proteingehalt wurde für dieses gereinigte VAC-Präparat ein Extinktionskoeffizient von

$A_{1cm}^{1\%}$ = 8,5 berechnet.

Charakterisierung des VAC

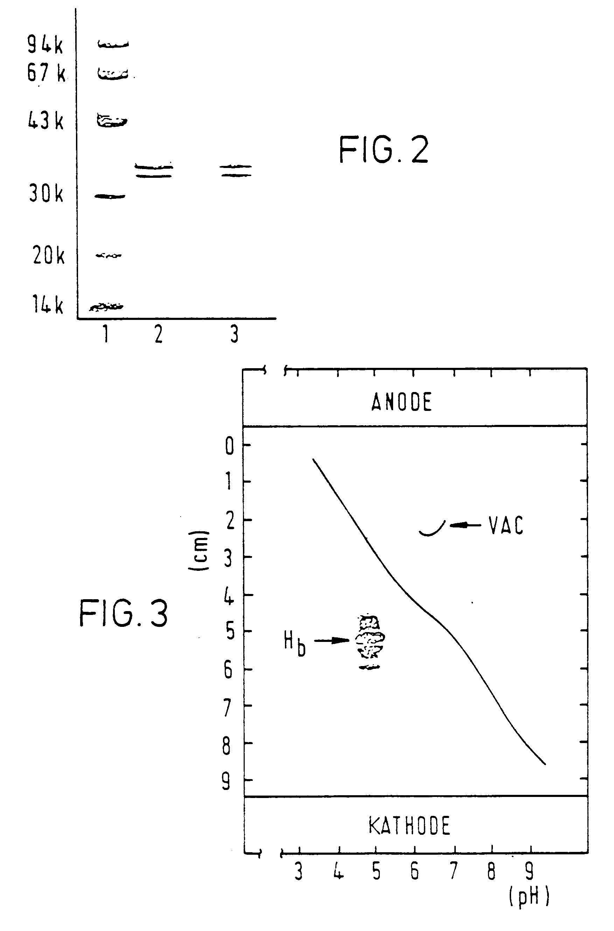

Wie aus Figur 2 hervorgeht, sind die zwei Polypeptide mit dem Molekulargewicht von 34.000 und 32.000, die in dem gereinigten Proteinmaterial vorhanden sind und denen die VAC-Aktivität zugeschrieben werden, Proteine mit einer einzigen Kette. Wie mit dem Schiff'schen Reagenz mit basischem Fuchsin festgestellt wurde, enthalten beide Proteine wenig Kohlehydratgruppen. Außerdem konnten in beiden Proteinen keine Gla-Gruppen festgestellt werden. Aus der isoelektrischen Fokussierung geht hervor, daß beide Proteine in einem einzigen Band wandern, was einem isoelektrischen Punkt von 4,4 bis 4,6 entspricht (vgl. Figur 3).

Die VAC-Aktivität wurde aus der PAG-Platte durch die Elution dieses Bandes aus dem Gel gewonnen. Eine Analyse dieses Eluates mit SDS-PAGE zeigte wiederum die Gegenwart der beiden Proteine. Damit konnte bestätigt werden, daß beide Proteine in einem einzigen Band in dem pH-Gradienten der PAG-Platte wandern. Zur Überprüfung der Methode wurde humanes Hämoglobin (Hb) ebenfalls mit Hilfe der isoelektrischen Fokussierung untersucht. Der festgestellte Wert von 6,8 stimmt mit den Angaben in der Literatur überein (vgl. Figur 3).

Aus Bindungsversuchen ging hervor, daß sich die VAC-Aktivität an negativ geladenen Phospholipid-Membranen binden kann. Diese Bindung erfolgt in Gegenwart von $Ca^{2+}$ und $Mn^{2+}$, aber nicht in Gegenwart von $Mg^{2+}$ und in Abwesenheit von divalenten Metallionen (vgl. Tabelle D). Diese Bindung der VAC-Aktivität an den Liposomen ist umkehrbar und kann durch das Reagenz EDTA aufgehoben werden.

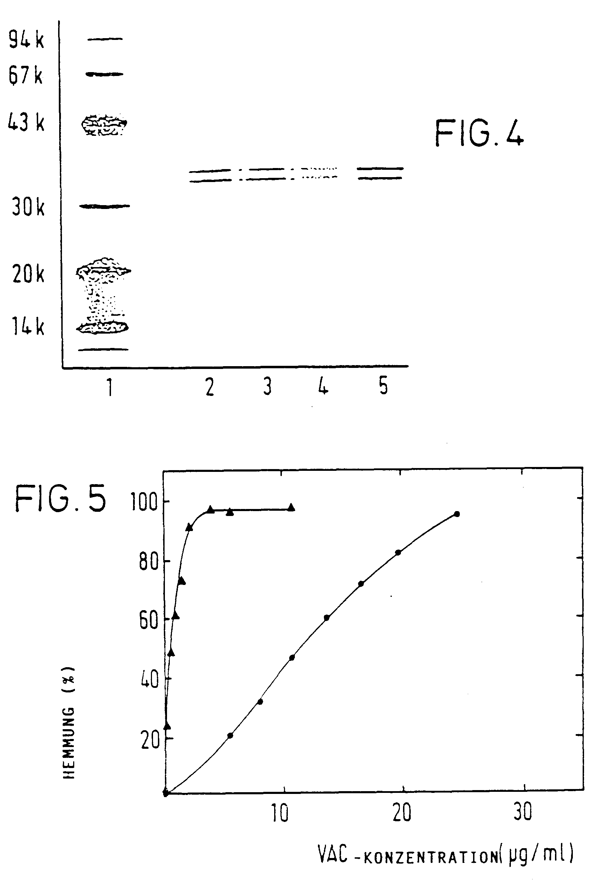

An Hand der SDS-PAGE konnte gezeigt werden, daß beide Proteine in Gegenwart von $Ca^{2+}$ an Liposomen binden können und daß diese Bindung bei Hinzufügen von EDTA wieder dissoziiert (vgl. Figur 4) ; ein erneuter Hinweis darauf, daß die VAC-Aktivität diesen zwei Proteinen zuzuschreiben ist.

Beim Aufbewahren in TBS, das 10 % Glycerol enthält, ist die VAC-Aktivität wenigstens 3 Monate bei -70 °C stabil, bei 0 °C wenigstens 12 Stunden und bei 37 °C wenigstens eine halbe Stunde. Bei 56 °C ist die Aktivität innerhalb von 2 Minuten verschwunden.

Wirkung von VAC

VAC verlängert die Gerinnungszeit nach dem Einstufenkoagulationsversuch, wobei die Koagulation mit BTP ausgelöst wird. Der Ersatz von BTP durch das gereinigte Rinderthrombin oder den gereinigten Rinderfaktor $X_a$ in diesem Versuch zeigt, daß VAC die Gerinnungszeit nur verlängert, wenn der Faktor $X_a$ zur Auslösung der Koagulation verwendet wird und daß die von Thrombin induzierte Gerinnung nicht durch VAC beeinflußt wird. Dies deutet darauf hin, daß VAC die Faktor $X_a$-Aktivität direkt hemmt oder daß eine Interaktion mit dem Prothrombinase-Komplex auftritt. Zur weiteren Prüfung wurde der amidolytische Thrombin-Bildungsversuch mit dem gereinigten Rinderfaktor $X_a$ und Prothrombin benutzt. Figur 5 zeigt, daß wenn Prothrombin in Gegenwart von $Ca^{2+}$ und Phospholipid durch Faktor $X_a$ zu Thrombin aktiviert wird, VAC die Prothrombin-Aktivierung hemmt und daß der Inhibitionsgrad von der VAC Konzentration abhängig ist. Außerdem ist der hemmende Effekt von VAC stärker bei einer niedrigeren Phospholipidkonzentration.

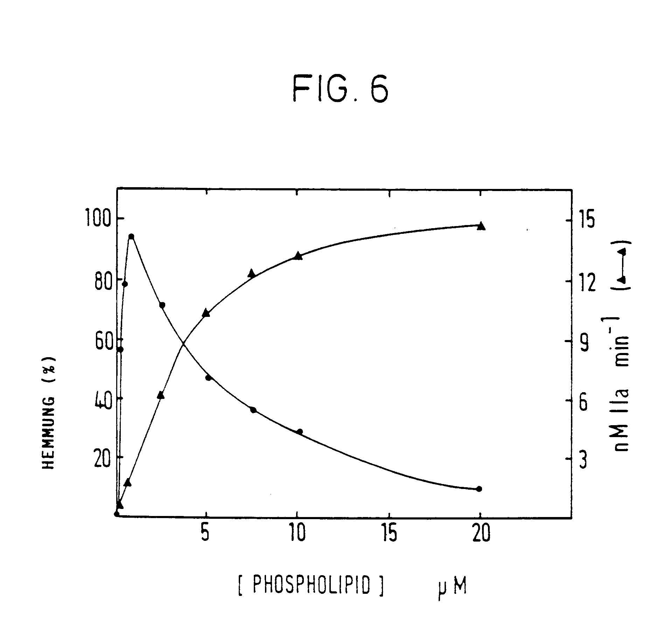

Figur 6 stellt die Phospholipidabhängigkeit der von VAC induzierten Inhibition der Prothrombin-Aktivierung dar. Bemerkenswert ist, daß bei einer Phospholipidkonzentration von Null die Thrombin-Aktivierung durch Faktor $X_a$ nicht von VAC gehemmt wird. Bei Kontrollversuchen zeigte sich, daß VAC selbst das Messsystem nicht beeinflußt.

Die Inkubation von 5 $\mu$M Phospholipid (PS/PC ; 1 : 4 Mol/Mol) mit 107 $\mu$g/ml VAC (spez. Akt. 1.300 Einheiten/mg) und 10 mM $Ca^{2+}$ ergab innerhalb von 3 Minuten bei 37 °C eine Verringerung der Prokoagulanz-Aktivität. Dies zeigte, daß VAC keine Phospholipase-Aktivität besitzt.

Im Gegensatz zu AT-III hat VAC keinen Einfluß auf die amidolytische Aktivität des gereinigten Thrombins und keinen nachhaltigen Einfluß auf die Faktor $X_a$-Aktivität, wie mit den chromogenen Substraten S 2238 beziehungsweise S 2337 gemessen wurde (vgl. Tabelle C). In dieser Tabelle wird auch gezeigt, daß die Inaktivierung von Faktor $X_a$ und Thrombin/AT-III nicht durch VAC verstärkt wird ; Heparin dagegen erhöht die AT-III Aktivität entscheidend. Dies zeigt, daß VAC weder eine Heparin-artige noch eine AT-IIIartige Aktivität besitzt.

Die Isolierung eines neuen menschlichen blutgerinnungshemmenden Proteins konnte durch ein prinzipiell gleiches Verfahren aus dem Homogenat von menschlichen Nabelschnurarterien erreicht werden.

Aufgefunden wurde das Antikoagulant durch seine Fähigkeit, die Gerinnungszeit in einem Prothrombin-zeitversuch zu verlängern. Diese Fähigkeit wurde nach einer Sephadex G-100 Fraktionierung eines Arterien-Homogenates meßbar. Aufgrund weiterer Isolierungsstufen kann angenommen werden, daß die Aktivität auf eine oder mehrere wasserlösliche Substanzen zurückgeführt werden kann, die insgesamt eine negative Ladung bei einem pH von 7,9 tragen.

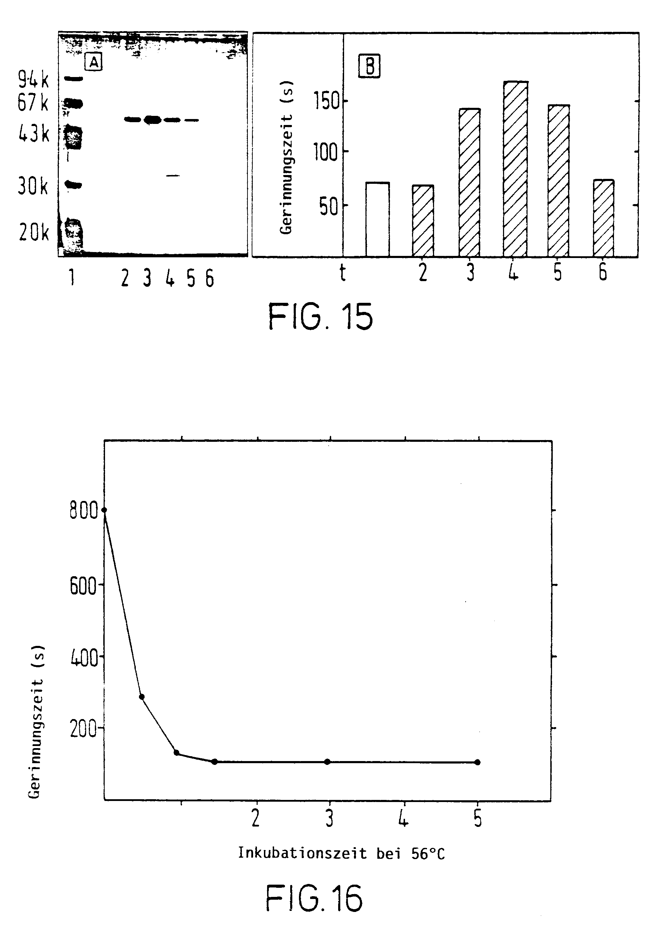

Die Analyse einer Sephadex G-75 Fraktion (der letzte bisher durchgeführte Reinigunsschritt) mit Hilfe der Gel-Elektrophorese ziegt eine Korrelation zwischen der Intensität der Molekulargewichtsbande bei 32.000 und der Verlängerung der Gerinnungszeit gemessen in dem MPTT (Figur 15). Die Beziehung zwischen der 32K-Bande und der Antigerinnungsaktivität ist zweifelsfrei, da nur aus diesem 32K-Band des Polyacrylamidgels eine blutgerinnungshemmende Aktivität eluiert werden kann. In Verbindung mit den Ergebnissen, daß das Antikoagulant seine Aktivität bei der Inkubation bei 56 °C schnell verliert und daß Proteasen seine Aktivität zerstören kann, muß angenommen werden, daß die Antigerinnungsaktivität durch ein einzelnes Protein mit einem offensichtlichen Molekulargewicht von 32.000 Dalton hervorgerufen wird.

Trypsin, im Gegensatz zu Protease Typ 1 ist ein schlechter Inaktivator des Antikoagulantes. Dies deutet darauf hin, daß das Antikoagulant nur wenige Lysin- und Arginin-Reste enthält, die für Trypsin zugänglich sind.

Die Art der blutgerinnungshemmenden Aktivität wurde untersucht, indem die Gerinnung auf verschiedene Weise ausgelöst wurde. Die entweder durch das vaskuläre Prokoagulanz HTP oder durch den Faktor $X_a$ ausgelöste Gerinnung wird durch das Antikoagulant gehemmt, die durch Thrombin induzierte Gerinnung jedoch nicht. Hieraus kann geschlossen werden, daß das Antikoagulant bei der Thrombinbildung nicht jedoch bei der Wirkung des Thrombins eine Rolle spielt.

Zur weiteren Aufklärung des Antigerinnungsmechanismus des erfindungsgemäßen Proteins wurde Prothrombinase verwendet (2). Unter den experimentellen Bedingungen kann das Antikoagulant die Aktivierung von Prothrombin durch die vollständige Prothrombinase (Faktor $X_a$, Faktor $V_a$, Phospholipid, $Ca^{2+}$) und durch den Phospholipid-gebundenen Faktor $X_a$ (Faktor $X_a$, Phospholipid, $Ca^{2+}$) hemmen, nicht aber den freien Faktor $X_a$ (Faktor $X_a$, $Ca^{2+}$).

Der Zeitverlauf der Prothrombinaktivierung in Gegenwart des Antigerinnungsmittels weist auf eine sofortige Inhibition der Prothrombinaktivierung hin, die zeitlich konstant bleibt. Es kann daher festgestellt werden, daß das Antikoagulant weder durch eine Phospholipase- noch durch eine proteolytische Aktivität wirkt. Die Tatsache, daß die Aktivierung von Prothrombin durch Faktor $X_a$ und $Ca^{2+}$ gar nicht von dem Antikoagulant beeinflußt wird, ist ein starker Hinweis darauf, daß der Antigerinnungsmechanismus des vaskulären Proteins von dem der allgemein bekannten Plasmaprotease-Inhibitoren, wie Antithrombin III verschieden ist. Wie von Walker et al. (8) gezeigt wurde, hemmt das aktivierte Protein C die Prothrombin-Aktivierung durch Faktor $X_a$, $Ca^{2+}$ und Phospholipid nicht ; demzufolge ist das erfindungsgemäße Protein auch nicht mit Protein C identisch.

Erste Bindungsversuche deuten darauf hin, daß das vaskuläre Antigerinnungsmittel mit der Lipidbindung des Faktors $X_a$ und/oder Prothrombins möglicherweise interferiert. Ob die Fähigkeit des Antikoagulantes, die Prothrombin-Aktivierung zu hemmen, vollständig für den Verlängerungseffekt im Prothrombin-Zeit-Versuch verantwortlich ist, muß noch ermittelt werden.

Die Tatsache, daß das erfindungsgemäße Material aus verschiedenen Arten von Arterien zu erhalten ist und nicht aus mäßig vaskularisiertem Gewebe, könnte darauf hinweisen, daß das erfindungsgemäße Protein ein physiologischer Modulator der Hämostase und Thrombose ist, aktiv auf dem vaskulärem Niveau.

Aufgrund der ermittelten Eigenschaften und Wirkungen des VAC kann der Blutgerinnungsmechanismus unter VAC Einfluß folgendermaßen interpretiert werden :

VAC bindet über $Ca^{2+}$-Ionen mit negativ geladenen Phospholipiden, die durch die Beschädigung von Gewebe und/oder die stimulierten Blutplättchen auftreten und verringert dadurch die Bindung bestimmter Gerinnungsfaktoren (Vitamin K abhängige Gerinnungsfaktoren) mit der negativ geladenen Phospholipidoberfläche, die für diese Gerinnungsfaktoren wie eine katalytische Oberfläche wirkt (Biochem. Biophys. Acta, 515, 163-205 (1978)), mit der Folge, daß die Phospholipid-abhängigen Blutgerinnungsreaktionen von VAC gehemmt werden. Aufgrund des Wirkungsmechanismus kann VAC in die Proteingruppe 3 eingeordnet werden (s. oben).

Von entscheidender Bedeutung ist jedoch der zu den Proteinen dieser Gruppe bestehende Unterschied, daß VAC die Phospholipide nicht hydrolisiert und dadruch keine wesentlichen Membranstrukturen zersetzen kann !

Weiterhin sind besonders bedeutsam und vorteilhaft die bisher bei keinem der bekannten Antikoagulanzien beschriebenen Eigenschaften des VAC :

Der Antigerinnungseffekt von VAC ist von der Menge Phospholipide abhängig, die an dem Gerinnungsprozeß teilnehmen. Diese Abhängigkeit bewirkt, daß der Gerinnungsprozeß, der beispielsweise durch eine

geringe Gefäßwandbeschädigung und/oder eine geringe Blutplättchenaktivierung ausgelöst wurde, z. B. durch einen thrombotischen Prozeß, überraschenderweise durch VAC gehemmt werden kann ! Andererseits wird jedoch der Gerinnungsprozeß, der durch eine drastische Gefäßwandbeschädigung ausgelöst wurde, bei der Phospholipide in hoher Konzentration auftreten, durch eben diese hohen Phospholipidkonzentrationen durch VAC nicht gehemmt ! Die Gefahr einer Blutung bei der Verwendung von VAC ist daher überraschenderweise äußerst gering.

Diese überlegenen Eigenschaften bietet VAC im Gegensatz zu allen bisher bekannten Antikoagulanzien, die einen oder mehrere Gerinnungsfaktoren unwirksam machen und dadurch die Gefahr einer Blutung erhöhen !

VAC inaktiviert überraschenderweise nicht die Gerinnungsfaktoren selbst ! Hierdurch ist es den Gerinnungsfaktoren auch weiterhin möglich, ihre anderen Aufgaben, von denen immer mehr aufgedeckt werden, zu erfüllen. Manche aktiven Gerinnungsfaktoren haben beispielsweise die wichtige nichthämostatische Aufgabe der Chemotaxis für die Inflammatorzelle. Diese Zelle trägt zur Heilung einer beschädigten Gefäßwand bei !

Durch VAC wird dieser Prozeß überraschenderweise nicht beeinträchtigt !

Die vorliegende Erfindung beschreibt erstmals eine neuartige Klasse von blutgerinnungshemmenden Proteinen, die die Gerinnungsfaktoren nicht inaktivieren. Die zur Erläuterung dienenden Beispiele sowie die aufgeführten Eigenschaften sollen die Erfindung in keiner Weise einschränken. Dem Fachmann ist es ohne erfinderische Leistung möglich, anhand des beschriebenen Verfahrens weitere Proteine, die blutgerinnungshemmende Eigenschaften aufweisen ohne die Gerinnungsfaktoren zu inaktivieren zu erhalten. Auch diese Proteine gehören zum Schutzumfang der vorliegenden Erfindung.

Die in der vorliegenden Erfindung verwendeten Abkürzungen haben folgende Bedeutung :

VAC : vaskuläres Antikoagulanz

PFP : Blutplättchenfreies Plasma

TBS : 100 mM NaCl, 50 mM Tris/HCl, pH 7.5

EDTA : Ethylendiamintetraessigsäure

TBSE : TBS mit 2 mM EDTA

BTP : Thromboplastin aus Rindergehirnen

HTP : Thromboplastin aus humanen Gehirnen

TBSA : TBS mit 0,5 mg/ml humanes Serum-Albumin, pH 7,9

S 2337 : N-Benzoyl-L-Isoleucyl-L-Glutamyl-L-Pipecolyl-Glycyl-L-Arginin-p-Nitroanilid-Dihydrochlorid

S 2238 : H-D-Phenylalanyl-L-Pipecolyl-L-Arginin-p-Nitroanilid-Dihydrochlorid

AT-III : humanes Antithrombin III

S.A. : spezifische Aktivität

$Ole_2$Gro-P-Cho : 1,2-Dioleoyl-sn-glycero-3-phospocholin

$Ole_2$Gro-P-Ser : 1,2-Dioleoyl-sn-glycero-3-phosposerin

Gla : $\gamma$-Carboxy-glutaminsäure

Für die Nomenklatur der Blut-Koagulationsfaktoren ist die von der Task Force on Nomenclature of Blood Clotting Zymogens and Zymogens intermediates empfohlene verwendet worden.

Materialien

Die Chemikalien für die analytische SDS-PAGE und Hydroxyapatit HTP stammten von Bio-Rad, Sephadex G-100 und G-75, DEAE-Sephacel und der « Low Molecular Weight Calibration Kit » von Pharmacia, die chromogenen Substrate S 2337 und S 2238 von Kabi Vitrum und die Diaflo PM-10 Ultrafiltrationsmembran von Amicon.

Beispiel I

Isolierung und Reinigung des VAC

Innerhalb einer halben Stunde nach dem Schlachten wurden den Rindern die Aorten entnommen. Das Rinderblut wurde in Trinatriumzitrat (Endkonzentration 0,38 Gew.%) gesammelt und 10 Minuten bei Raumtemperatur und 2.000 xg zentrifugiert. Das wenige Blutplättchen enthaltende Plasma wurde anschließend erneut zentrifugiert (15 Minuten bei 10.000 xg). Auf diese Weise erhielt man blutplättchenfreies Plasma (PFP).

Die Aorten der Tiere wurden unmittelbar nach ihrer Entnahme gründlich mit TBS gespült. Die Intima wurden von den Aorten gelöst und unter Anwendung eines hochtourigen Homogenisators, beispielsweise mit dem Braun MX 32, in TBSE, das Sojabohnen-Trypsin-Inhibitor (16 mg/l) und Benzamidin (1,57 g/l) enthielt, homogenisiert.

Das aus 8 Aorten homogenisierte Material, das 20 % (Gewicht/Volumen) Feststoff enthielt, wurde 60 Minuten bei 100.000 xg zentrifugiert. Die überstehende Flüssigkeit wurde mit festem Ammoniumsulfat bis zu 30 % gesättigt, 30 Minuten gerührt und anschließend 20 Minuten bei 12.000 xg zentrifugiert. Die resultierende Flüssigkeit wurde mit festem Ammoniumsulfat bis zu 90 % gesättigt, 30 Minuten gerührt und anschließend 20 Minuten bei 12.000 xg zentrifugiert. Der Feststoff wurde in einem kleinen Volumen TBS suspendiert und gegen Benzamidin (1,57 g/l) enthaltendes TBS dialysiert. Die dialysierte Fraktion wurde auf eine Hydroxyapatit-Säule (1 × 20 cm) aufgetragen, die mit TBS äquilibriert worden war. Die Säule wurde mit 4 Bettvolumina TBS gewaschen. Die VAC-Proteine wurden mit 200 ml Natriumphosphatpuffer (pH 7,5) mit einem Lineargradienten von 0-500 mM aus der Säule eluiert. Die VAC enthaltenden Fraktionen wurden vereinigt und gegen 50 mM NaCl, 20 mM Tris/HCl, pH 7,5 dialysiert.

Der gleiche Puffer wurde auch zur Äquilibrierung einer DEAE-Sephacel-Säule (3 × 5 cm) verwendet, auf der das dialysierte VAC-Material chromatographiert wurde. Die Säule wurde mit 4 Bettvolumina des Äquilibrationspuffers gewaschen bevor das VAC mit 200 ml NaCl-Lösung in 20 mM Tris/HCl, pH 7,5 mit einem Lineargradienten von 50-300 mM von der Säule eluiert wurde. Die Vac enthaltenden Fraktionen wurden gesammelt. gegen 500 mM NaCl, 20 mM Tris/HCl, pH 7,5 dialysiert und danach in einer Amicon-Konzentrationszelle unter Verwendung einer PM-10 Ultrafiltrationsmembran konzentriert. Das Konzentrat mit einem Volumen von 2 ml wurde auf eine Sephadex G-100 Säule (3 × 80 cm), die mit 500 mM NaCl und 20 mM Tris/HCl, pH 7,5 äquilibriert worden war, aufgetragen.

Das Eluat wurde in Fraktionen zu je 2 ml gesammelt, die aktiven Fraktionen gesondert gegen 10 Volumen-% Glycerol enthaltendes TBS dialysiert und bei - 70°C aufbewahrt. Die ganze Reinigung wurde bei 0-4 °C durchgeführt.

Bestimmung der VAC-Aktivität

Zur Bestimmung der VAC-Aktivität wurden zwei verschiedene Methoden verwendet :
a) der Einstufenkoagulationsversuch (modifizierter Prothrombinzeitversuch)
b) ein Thrombinbildungsversuch
Der Einstufenkoagulationsversuch wurde folgendermaßen ausgeführt :

In einer Küvette aus silikonisiertem Glas wurden 175 $\mu$l der zu prüfenden Fraktion oder 175 $\mu$l TBS als Kontrolle mit 50 $\mu$l PFP und 25 $\mu$l verdünntem BTP gerührt (900 Umdrehungen/Min.).

Nach einer Inkubationszeit von 3 Minuten bei 37 °C wurde die Koagulation durch das Hinzufügen von 250 $\mu$l Puffer, der 80 mM NaCl, 20 mM $CaCL_2$ und 10 mM Tris/HCl, pH 7,5 enthielt, ausgelöst. Die Fibrin-Bildung wurde optisch unter Verwendung einer ≪ Payton Dual Aggregation Module ≫ (Hornstra, G., Phil. Trans. R. Soc. London B. 294, 355-371 (1981) registriert. Die Gerinnungszeit der Kontrollprobe betrug 65 Sekunden. Dieser Versuch wurde während der Reinigung zur Prüfung der verschiedenen Fraktionen auf das Vorhandensein der VAC-Aktivität angewandt. Zur Bestimmung der VAC-Ausbeute während der Reinigung wurde eine Einheit der VAC-Aktivität definiert als jene Menge VAC, die die Gerinnungszeit in dem obenerwähnten Versuch bis 100 Sekunden verlängerte.

In einigen Fällen wurde BTP durch gereinigtes Rinderthrombin oder den gereinigten Rinderfaktor $X_a$ ersetzt. In diesem halbgereinigten Gerinnungssystem wurden die Mengen Thrombin oder Faktor $X_a$ derart gewählt, daß die Gerinnungszeit der Kontrollprobe ebenfalls 65 Sekunden betrug.

Der Thrombinbildungsversuch wurde folgendermaßen ausgeführt :

20 $\mu$l gereinigter Rinderfaktor $X_a$ (150 nM), 30 $\mu$l $CaCL_2$ (100 mM), 30 $\mu$l verdünnter VAC und 30 $\mu$l PS/PC-Phospholipid-Membrane (die Endkonzentrationen sind in der Legende zu den Abbildungen angegeben) wurden in eine Kunststoffküvette, die 181 $\mu$l TBSA enthielt, gebracht.

Das Gemisch wurde 3 Minuten bei 37 °C mit einem Teflonrührer gerührt. Die Thrombinbildung wurde durch Hinzufügung von 9 $\mu$l gereinigtem Rinderfaktor II (33,33 $\mu$M) ausgelöst. Nach verschiedenen Zeiten wurden dem Reaktionsgemisch Proben von 50 $\mu$l entnommen, die dem Inhalt einer Kunststoffküvette von 1 ml, die mittels eines Thermostaten auf 37°C temperiert wurde und 900 $\mu$l TBSE und 50 $\mu$l des chromogenen Substrats S 2238 (5 mM) enthielt, hinzugefügt wurden. Aus der Extinktionsänderung bei 405 nm, mit einem Kontron Spektrometer Uvikon 810 gemessen, wurde die Menge Thrombin in dem Reaktionsgemisch unter Verwendung einer Eichkurve, die aus den bekannten Mengen gereinigten Rinderthrombins erstellt worden war, berechnet. Der Prozentsatz der von VAC verursachten Inhibition wurde folgendermaßen definiert :

% Inhibition = (1 - a/b) × 100 %,

worin ≪ a ≫ die Geschwindigkeit der Thrombinbildung in Anwesenheit von VAC in mN II$_a$/Min. und ≪ b ≫ die Geschwindigkeit der Thrombinbildung in Abwesenheit von VAC in nM IIa/Min. ist.

Proteine

Die von Vitamin K abhängigen Faktoren Prothrombin und Faktor X$_a$ wurden durch die Reinigung des zitrierten Rinderplasmas erhalten (vgl. Stenflo J.; J. Biol. Chem. 251 355-363 (1976)). Nach der Bariumzitrat-Adsorption und Elution, Fraktionierung mit Ammoniumsulfat und DEAE-Sephadex-Chromatographie gab es zwei Proteinfraktionen, die eine Prothrombin/Faktor IX Mischung, beziehungsweise Faktor X enthielten. Factor X wurde nach der Methode von Fujikawa et al., Biochemistry, 11, 4882-4891 (1972) erhalten und unter Anwendung von RVV-X (Fujikawa et al., Biochemistry, 11, 4892-4899 (1972)) aktiviert. Prothrombin wurde von Faktor IX durch eine Heparin-Agarose-Affinitätschromatographie (Fujikawa et al., Biochemistry, 12, 4938-4945)) abgetrennt. Die Prothrombin enthaltenden Fraktionen aus der Heparin-Agarose-Säule wurden vereinigt und weiter nach der Methode von Owens et al., J. Biol. Chem. 249, 594-605 (1974)) gereinigt. Die Konzentrationen von Prothrombin und Faktor X$_a$ wurden nach der Methode von Rosing et al., J. Biol. Chem. 255, 274-283 (1980) bestimmt. BTP wurde nach einer Normalmethode hergestellt, die von Van Dam-Mieres et al. in ≪ Blood coagulation enzymes, Methods of Enzymatic Analysis ≫ Verlag Chemie GmbH, Weinheim beschrieben ist. Die Proteinkonzentrationen wurden nach Lowry et al., J. Biol. Chem. 193, 265 (1951) bestimmt.

Herstellung von Phospholipid, Phospholipid-V esikel und Phospholipid-Liposom

1,2-Dioleoyl-sn-Glycero-3-Phosphocholin (18 : 1$_{cis}$/18 : 1$_{cis}$-PC) und 1,2-Dioleoyl-sn-Glycero-3-Phosphose-rin (18 : 1$_{cis}$/18 : 1$_{cis}$-PS) wurden wie bei Rosing et al., J. Biol. Chem. 255, 274-283 (1980) beschrieben hergestellt. Gesonderte, aus zwei Schichten bestehende Phospholipid-Vesikel aus PC und PS wurden unter Anwendung von Ultraschall hergestellt, wie es bei Rosing et al., J. Biol. Chem. 225, 274-283 (1980) beschrieben ist. Eine Vorratslösung aus Phospholipid-Liposomen wurde durch Lösen der erforderlichen Menge an Phospholipid in Chloroform hergestellt. Das Chloroform wurde mittels Stickstoff verdampf. Das zurückbleibende Phospholipid wurde in 5 % Glycerol enthaltendem TBS suspendiert, 3 Minuten vorsichtig mit einigen Glaskügelchen gemischt und danach 10 Minuten bei 10.000 xg zentrifugiert. Die obenstehende Lösung wurde verworfen und der Rückstand vorsichtig erneut in 5 % Glycerol enthaltendem TBS suspendiert ; auf diese Weise erhielt man die Phospholipid-Liposornen-Vorratslösung. Diese Liposomen wurden bei Raumtemperatur aufbewahrt. Die Phospholipid-Konzentrationen wurden durch die Phosphatanalyse nach Böttcher et al., Anal. Chim. Acta, 24, 203-207 (1961) bestimmt.

SDS-PAGE

Die Gel-Elektrophorese auf Platten in Gegenwart von SDS wurde nach der von Laemmli, Nature, 227, 680-685 (1970) beschriebenen Methode unter Verwendung eines Gels ausgeführt, das 10 Gew.% Acrylamid, 0,27 Gew.% N/N³-Methylen-bisacrylamid und 0,1 Gew.% SDS enthielt. 5 Gew.% $\beta$-Mercaptoethanol war in Gelproben mit reduzierten Disulfidbrücken vorhanden. Die Gele wurden folgendermaßen gefärbt :
1) mit 0,25 Gew.% Coomassie Blue R-250 in 50 Gew.% Ethanol und 15 Gew.% Essigsäure und entfärbt in 10 Gew.% Ethanol und 10 Gew.% Essigsäure,
2) mit Schiff'schem Reagenz, hergestellt aus dem basischen Fuchsin (Merck) nach der Methode von Segrest et al., beschrieben in ≪ Methods in Enzymology ≫, Vol. 28, 54-63 (1972) hergestellt oder
3) mit Silber wie von Merril et al. in Electrophoresis, 3, 17-23 (1982) beschrieben.

Elektrofokussierung

Die isoelektrischen pH-Bestimmungen von Proteinen wurden mit den fertigen Dünnschicht-Polyacrylamidgelen, die Amfolin Träger-Ampholyte enthalten (PAG-Platten, LKB) bei einem pH in dem Bereich von 3,5-9,5 nach Anleitung des Herstellers ausgeführt. Der pH-Gradient in dem Gel wurde sofort nach der Elektrofokussierung durch Abschneiden eines Streifens von dem Gel entlang einer Linie zwischen der Anode und der Kathode bestimmt. Die Elektrolyten wurden aus jedem Streifen mit destilliertem Wasser eluiert und der pH-Wert des Wassers mit einer kombinierten Glaselektrode gemessen.

γ-Carboxy-Glutaminsäurebestimmung

Die Gla-Bestimmung wurde mit Hilfe der HPLC an einer ≪ Nucleosil 5S B ≫-Säule (CHROMPACK) nach der Methode von Kuwada et al., Anal. Biochem. 131, 173-179 (1983) ausgeführt.

Beispiel II

Die Kupplung von Phospholipiden an Sphérocil

Die erforderlichen Phospholipide wurden in Chloroform gelöst und dem Säulenmaterial Sphérocil (Firma Rhone-Poulenc) in einem Verhältnis von 5 mg Phospholipid pro Gramm Sphérocil hinzugefügt. Das Chloroform wurde mit $N_2$-Gas verdampft und das trockene Sphérocil-phospholipid anschließend mit dem Puffer gewaschen, worin VAC suspendiert worden war. VAC bindet an Sphérocil-Phospholipid in Gegenwart von $Ca^{++}$ und/oder $Mn^{++}$, wenn ein Teil der Phospholipide negativ geladen ist.

Beispiel III

Es wurde 50 µl zitriertes/Plättchen-freies Plasma gemischt mit 200 µl Puffer (25 mM Tris/HCl pH 7,5, 100 mM NaCl), in dem Kaolin (artifizielle Oberfläche, die die wesentliche Gerinnung katalysiert, in diesem Falle gemahlenes Glas), Inositin (Phospholipid-Quelle) und VAC vorhanden waren. Dieses Gemisch wurde 3 Minuten bei 37 °C inkubiert, wonach 250 µl $Ca^{++}$-Puffer (200 mM Tris/HCl pH 7,5, 80 mM NaCl, 20 nM $CaCl_2$) hinzugefügt wurden. Die Gerinnungszeit wurde wie in Beispiel I gemessen.

Tabelle A

| Zusammenfassung der Reinigung von VAC aus Intima der Rinderaorten | | | | | |
|---|---|---|---|---|---|
| Reinigungsstufe | Protein[a] mg | VAC[b] Einheiten | Spezifische Aktivitäten Einheiten/mg | Ausbeute % | Reinigungsgrad |
| Obenstehende Flüssigkeit mit 35 % $(NH_4)_2SO_4$ | 630 | 19.500 | 31,0 | 100 | 1,0 |
| Präzipitat mit 90 % $(NH_4)_2SO_4$ | 470 | 19.000 | 40,4 | 97 | 1,3 |
| Hydroxy apatit Fraktion | 206 | 17.300 | 84,0 | 89 | 2,7 |
| DEAE-Fraktion | 35,8 | 13.900 | 388 | 71 | 12,5 |
| Sephadex G-100 Fraktion 139 | 0,45 | 666 | 1 480 | 3,4 | 47,7 |

a) Die Menge Protein wurde nach der Methode von Lowry et al. bestimmt (Lowry, O.H., Rosebrough N.V., Farr A.L. und Randall R.J. J. Biol. Chem. 193 (1951) 265).
b) Die VAC-Einheiten wurden bestimmt nach dem in Beispiel I beschriebenen Einstufenkoagulationsversuch an einer Reihe Probeverdünnungen. Jene Verdünnung, welche die Gerinnungszeit der Kontrollprobe (65 Sek.) zu 100 Sek. verlängert, enthält nach der Definition eine VAC-Einheit.

Tabelle B

| Die Metall-abhängige Bindung von VAC an negativ geladene Phospholipid-Liposomen | | |
|---|---|---|
| Kation (10 mM) | $t_c$, Sekunden[a] obenstehende Flüssigkeit | EDTA[c] |
| Kontrolle (keine Liposomen) | 180 | N.D.[d] |
| Kontrolle (kein Kation) | 174 | 64,8 |
| CaCl$_2$ | 64,2 | 134 |
| MgCl$_2$ | 165 | N.D. |
| MnCl$_2$ | 65,1 | N.D. |

a) Die Gerinnungszeiten $t_c$ wurden nach dem in Beispiel I beschriebenen Einstufenkoagulationsversuch bestimmt.

b) 50 $\mu$l der Vorratslösung von Phospholipid-Liposomen (PS/PC ; 50/50 mol/mp : 1 mM), 50 $\mu$l VAC (250 $\mu$g/ml, S.A. = 700 Einheiten/mg) und 100 $\mu$l TBS, das 5 % Glycerol und das angegebene Kation enthielt, pH 7,5, wurden bei Zimmertemperatur vermischt und 15 Minuten bei 15 000 xg zentrifugiert, 25 $\mu$l der obenstehenden, erhaltenen Flüssigkeit wurde mit TBS bis 175 $\mu$l verdünnt und dann nach dem Einstufenkoagulationsversuch geprüft. Die Restmenge der obenstehenden Flüssigkeit wurde mit SDS-PAGE analysiert (Fig. 4).

c) Das erhaltene Liposom-Präzipität wurde aufs neue suspendiert in 150 $\mu$l 5 % Glycerol enthaltendes TBS und 10 mM EDTA, pH 7,5. Die Suspension wurde während 15 Minuten bei 15 000 xg zentrifugiert. Die VAC-Aktivität der obenstehenden Flüssigkeit wurde wie unter b) beschrieben, analysiert.

d) N.D. = nicht bestimmt.

Tabelle C

| Der Effekt von VAC auf die amidolytische Aktivität von Faktor $X_a$ und Faktor $II_a$ | | |
|---|---|---|
| | $A_{405}$/Min . $10^3$ a VAC | |
| | - | + |
| $X_a$ | 110.5 | 110.5 |
| $X_a$, AT-III | 80.0 | 81.5 |
| $X_a$, Heparin | 110.5 | 109.0 |
| $X_a$, Heparin, AT-III | 47.5 | N.D.[b] |
| $II_a$ | 7.5 | 7.5 |
| $II_a$, AT-III | 5.4 | 5.6 |
| $II_a$, Heparin | 7.5 | 7.1 |
| $II_a$, Heparin, AT-III | 0.56 | N.D. |
| Die Endkonzentrationen der verschiedenen Mittel in den Reaktionsgemischen waren wie folgt : 18,7 nM Faktor $X_a$ ; 1,5 nM Faktor $II_a$ ; 18,7 nM AT-III ; 1 Einheit/ml) Heparin und 10,7 $\mu$g/ml VAC (S.A. 1300 Einheiten/mg). b) N.D. = nicht bestimmt. | | |

a) Die amidolytische Aktivität wurde wie folgt gemessen : Faktor $X_a$ oder Faktor $II_a$ wurde mit den erwähnten Mitteln in TBSA verdünnt. Das Reaktionsgemisch wurde mit einem mit Teflon überzogenen Rührer in einer Kunststoffküvette, die mit Hilfe eines Thermostaten auf 37 °C temperiert wurde, gerührt. Nach 10 Minuten wurde eine Probe von 100 $\mu$l ($X_a$) oder 50 $\mu$l ($II_a$) aus der Küvette genommen. Diese Probe wurde in eine andere, mit Hilfe eines Thermostaten auf 37 °C temperierte Kunststoffküvette gebracht, welche 800 $\mu$l TBSE und 100 $\mu$l S 2337 (2mM) beziehungsweise 900 $\mu$l S 2238 (5 mM) enthielt. Die Absorptionsänderung bei 405 nM wurde mit einem Kontron Spectrophotometer Uvikon 810 gemessen, das mit Hilfe eines Thermostaten auf 37 °C temperiert wurde.

Beispiel IV

Das durch Venenpunktion gesammelte menschliche Blut wurde in einer Trinatriumzitratlösung (Endkonzentration etwa 13 mM Zitrat) gesammelt und 10 Minuten bei 2 000 xg und Raumtemperatur zintrifugiert. Das so erhaltene Plasma wurde erneut 15 Minuten bei 10 000 xg zintrifugiert, um blutplättchenfreies Plasma (PFP) zu erhalten. Als Standard dienendes PFP wurde durch Mischen von Plasma verschiedener gesunder Spender bereitet.

Menschliche Nabelschnüre wurden 15 Minuten nach der Geburt erhalten. Die Arterien wurden sofort mit eiskaltem TBS-Puffer gespült, danach von dem « Jelly von Warton » (Warton'sche Gel) freipräpariert und in TBS unter Verwendung eines Homogenisators des Typs Braun MX32 homogenisiert. Ein 10 % Homogenisat (w/v) wurde anschließend fraktioniert.

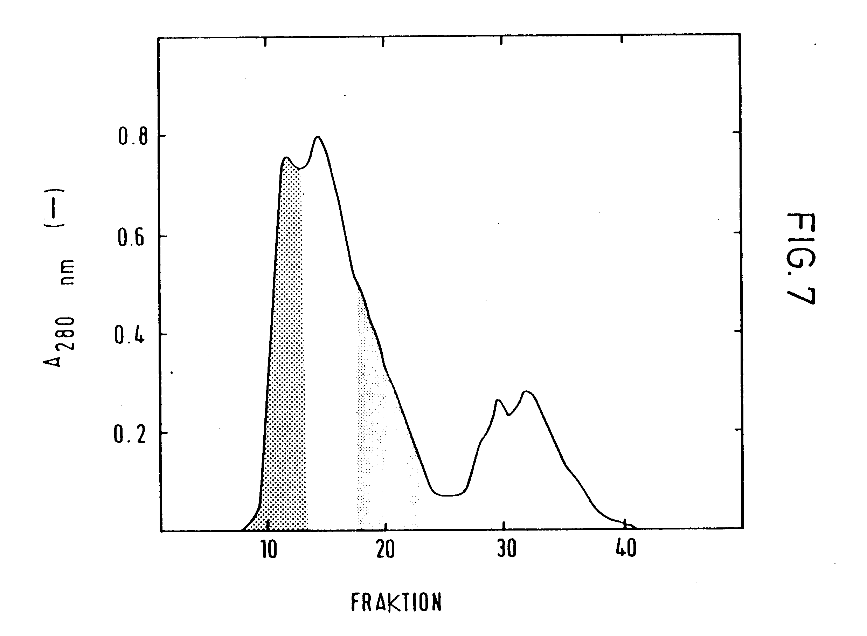

Die Fraktionierung des Überstandes des bei 10 000 xg zentrifugierten Homogenates mit Sephadex G-100 ergab ein reproduzierbares spezifisches Elutionsprofil (siehe Fig. 7).

Die Fraktionen, die das Gerinnungssystem bei der Messung in dem MPTT beeinflussen, sind in Fig. 7 dargestellt. Eine Prokoagulansaktivität eluierte mit dem Vorlauf. Diese Aktivität kann im MPTT nur in Gegenwart des Faktors VII nachgewiesen werden durch Versuche, bei denen menschliches Kongenitalfaktor-VII-defizientes Plasma verwendet wurde. Demzufolge muß dieser Prokoagulant als Gewebe-Thromboplastin betrachtet werden.

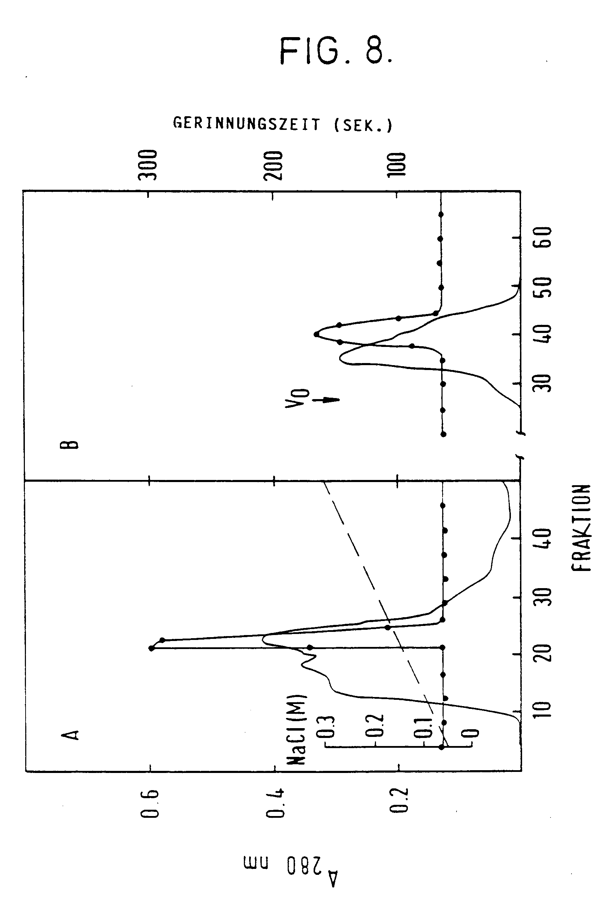

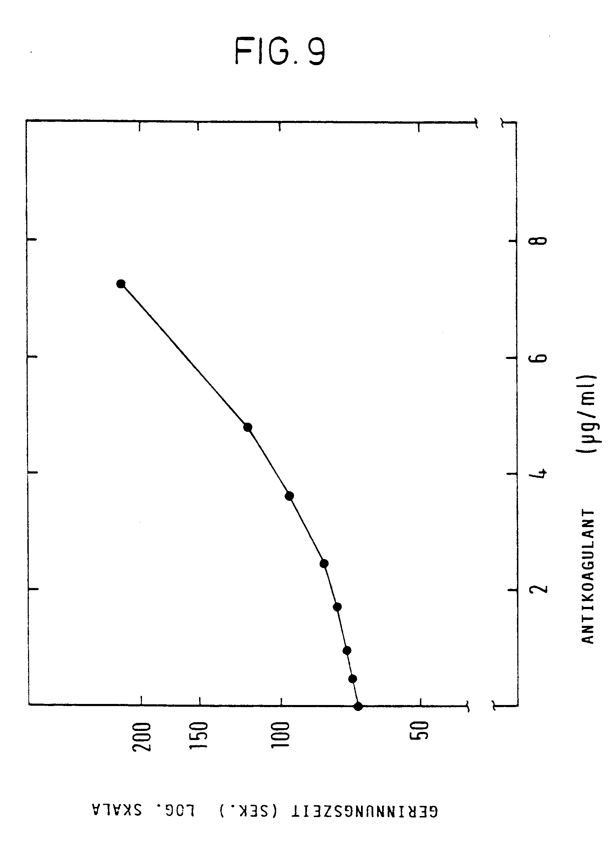

Bestimmte Fraktionen hatten eine ausgeprägte Antigerinnungsaktivität. Diese Fraktionen wurden vereinigt und durch DEAE-Sephacel-Chromatographie weiter gereinigt (Fig. 8A). Das Antikoagulant hatte sich an dem DEAE-Sephacel bei einer Salzkonzentration von 50 mM NaCl und 50 mM Tris/HCl pH 7,9 gebunden. Die Elution der Aktivität mit einem linearen NaCl-Gradienten bei pH 7,9 wurde mit 150-160 mM NaCl durchgeführt. Die DEAE-Fraktionen, die die Antikoagulansaktivität enthielten, wurden vereinigt und einer Gelfiltration mit Sephadex G-75 unterzogen. Die Säule (1,5 × 50 cm) wurde mit TBS äquilibriert. Die Aktivität trat in den Fraktionen mit einem Molekulargewicht von 30 000-60 000 Dalton auf. Der MPTT wurde als quantitativer Assay zur Bestimmung der Menge an Antikoagulant-Aktivität verwendet (Fig. 9). Eine Einheit Antikoagulant-Aktivität wurde definiert als die Menge, die die Gerinnungszeit im MPTT, bei Verwendung von HTP als Auslöser der Gerinnung (Endkonzentration 95 $\mu$g Protein/ml) von dem Kontrollwert von 65 sec. auf 100 sec. verlängert. Mit Hilfe dieses Assays wurde errechnet, daß aus 10 g nassem, arteriellem Gewebe etwa 2 mg Protein mit annähernd 1 200 Einheiten Antikoagulant-Aktivität erhalten wurde.

Modifizierter Prothrombin-Zeit-Versuch (MPTT)

Der modifizierte Prothrombin-Zeit-Versuch (MPTT) wurde folgendermaßen durchgeführt. In einer Küvette aus silikonisiertem Glas wurden 50 $\mu$l PFP bei einer Temperatur von 37 °C mit 150 $\mu$l TBS, 25 $\mu$l einer Norm HTP-Verdünnung und 25 $\mu$l TBS (Kontrolle) oder mit 25 $\mu$l einer Fraktion des homogenisierten Arterienmaterials gerührt. Nach einer Inkubation von 3 Minuten wurde die Gerinnung durch Hinzufügung von 250 $\mu$l $Ca^{2+}$-Puffer (80 mM NaCl, 20 mM $CaCl_2$ und 10 mM Tris/HCl pH 7,9) ausgelöst. Die Fibrinbildung wurde mittels einer ≪ Payton Dual Aggregation Module ≫ (13) registriert. Wurde in dem MPTT zur Auslösung der Gerinnung der Faktor $X_a$ benutzt, wurde HTP weggelassen und 25 $\mu$l $Ca^{2++}$-Puffer zum verdünnten PFP gegeben.

Modifizierter Thrombin-Zeit-Versuch (MTT)

Der modifizierte Thrombin-Zeit-Versuch (MTT) wurde auf die gleiche Weise ausgeführt wie oben beschrieben, mit dem Unterschied, daß das $X_a$-Präparat durch 25 $\mu$l gereinigtes Thrombin ersetzt wurde.

Proteine

Protease Typ I und Trypsin (EC 3.4.2.1.4) stammten von Sigma. HTP wurde nach der von Van Dam-Mieras et al. (14) beschriebenen Methode aus Menschengehirnen hergestellt. Faktor $X_a$, Prothrombin und Thrombin wurden aus zitriertem Rinderblut wie bei Rosing et al. (2) beschrieben, gereinigt. Faktor V wurde nach der bei Lindhout et al. (13) beschriebenen Methode aus Rinderblut erhalten. Faktor $V_a$ erhielt man durch Inkubation des Faktors V mit Thrombin (15). Die Prothrombin-Konzentration wurde berechnet aus $M_r$ = 72 000 und A 1 %/280 = 15,5 (16) ; die Faktor V-Konzentration wurde berechnet aus $M_r$ 330 000 und A 1 %/280 = 9,6 (17). Faktor $X_a$- und Thrombin-Konzentrationen wurden durch Titration der aktiven Stellen (2) bestimmt. Die übrigen Proteinkonzentrationen wurden bestimmt, wie bei Lowry et al. (18) beschrieben.

Herstellung von Phospholipiden und Phospholipid-Vesikel

$Ole_2$Gro-P-Cho und $Ole_2$Gro-P-Ser wurden nach der Methode von Rosing et al. (2) hergestellt. Durch Ultraschall wurden einzelne Doppelschicht-Vesikel aus $Ole_2$Gro-P-Ser/$Ole_2$Gro-P-Cho (Molverhältnis 20 : 80) hergestellt (2).
Die Phospholipidkonzentrationen wurden durch Phosphat-Analyse nach Böttcher et al. (19) bestimmt.

Messung der Prothrombin-Aktivierung

Der Zeitverlauf der Prothrombin-Aktivierung wurde mit verschiedenen Konzentrationen des Antigerinnungsmittels untersucht. Die Gemiche von ($X_a$, $Ca^{2+}$), ($X_a$, Phospholipid, $Ca^{2+}$) oder ($X_a$, $V_a$,Phospholipid, $Ca^{2+}$) wurden mit verschiedenen Mengen des Antigerinnungsmittels bei 37 °C in 50 mM Tris/HCl, 175 mM NaCl, 0,5 mg/ml Human-Serum-Albumin bei einem pH-Wert von 7,9 gerührt. Nach 3 Minuten wurde die Reaktion durch Hinzufügung von Prothrombin ausgelöst. Proben von 25 $\mu$l wurden in verschiedenen zeitlichen Abständen in eine Küvette eingebracht (auf 37 °C thermostatisiert), die TBS, 2 mM EDTA und 0,23 mM S2238 enthielt (das Gesamtvolumen betrug schließlich 1 ml). Aus der Absorptionsänderung bei 405 nm, gemessen mit einem Kontron Spectrophotometer Uvikon 810, und einer Messkurve, die anhand bekannter Mengen gereinigten Thrombins erstellt worden war, wurde die Menge des gebildeten Thrombins bei verschiedenen Antigerinnungsmittelkonzentrationen berechnet. Das Phospholipid wurde als Vesikel aus $Ole_2$Gro-P-Ser und $Ole_2$Gro-P-Cho mit einem Molverhältnis von 20 : 80 hinzugefügt.

Charakterisierung

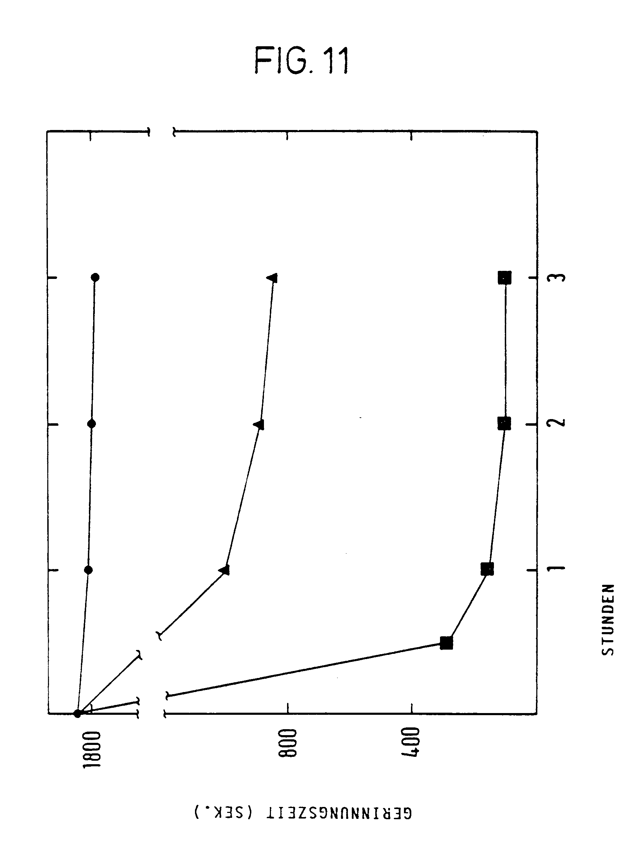

Verschiedene Fraktionen der Chromatograhie über G-75 wurden im MPTT geprüft und mit SDS-PAGE analysiert. Die Ergebnisse (siehe Fig. 10) deuten an, daß das Antigerinnungsmittel ein Molekulargewicht von etwa 32 000 besitzt. Die Beziehung zwischen der Antikoagulanzaktivität und der 32K-Bande wurden bestätigt, indem das Polyacrylamidgel geschnitten wurde und die Proteine anschließend aus dem Streifen mit TBS, das 0,5 mg/ml Rinderserum-Albumin enthielt, eluiert wurden. Eine Antikoagulant-Aktivität ist nur in dem Streifen vorhanden, der der 32K-Bande zuzuordnen ist. Außerdem ergab sich, daß diese Aktivität bei 56 °C thermolabil ist und eine ähnliche Dosis/Wirkung im MPTT aufweist, wie das Ausgangsmaterial. Die G-75 Fraktionen, die die höchsten Antigerinnungsaktivitäten zeigten, wurden vereinigt und für die weitere Kennzeichnung des Antigerinnungsmittels verwendet. Die Inkubation des Antigerinnungsmittels bei 56 °C ergibt eine rasche Erniedrigung der Aktivität bis nach 2 Minuten keine Aktivität mehr gemessen werden kann (siehe Fig. 16). Das Antigerinnungsmittel verliert seine Aktivität vollständig innerhalb von 2 Stunden bei der Inkubation bei 37 °C mit Protease Typ I, während Trypsin das Antigerinnungsmittel innerhalb einer Inkubationsperiode von 3 Stunden kaum inaktiviert (siehe Fig. 11). Die bei diesen Versuchen benutzte Konzentration an Protease Typ I und Trypsin inaktiviert 2,5 nM Thrombin innerhalb von 15 Minuten. Die Mengen an Protease Typ I und Trypsin, die von den Reaktionsgemischen in dem MPTT übertragen wurde, zeigen keinen Einfluß auf die Kontrollgerinnungszeit.

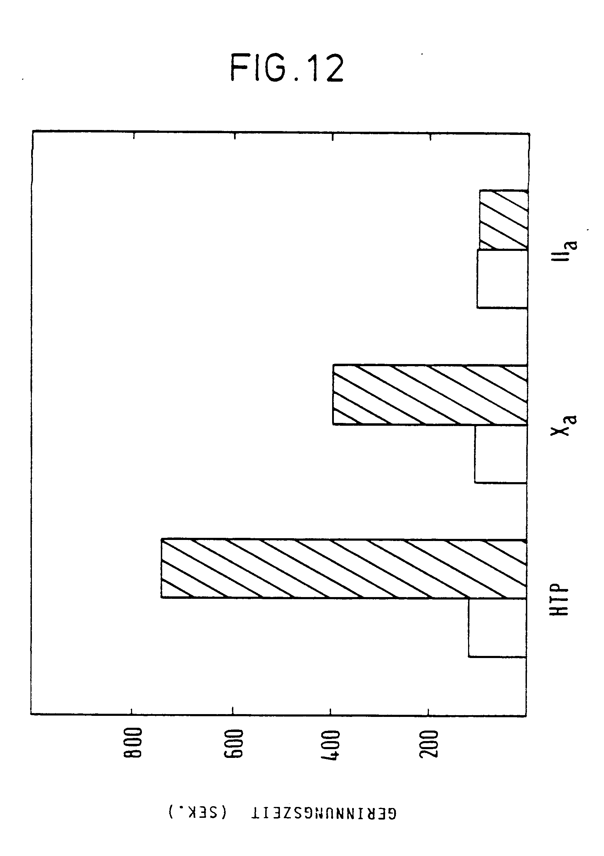

Der MPPT wird sowohl in Gegenwart des Antigerinnungsmittels (siehe Fig. 12) als auch bei der Aktivierung mit HTP und bei der Auslösung mit dem Faktor $X_a$ verlängert. Eine von Thrombin induzierte Gerinnung wird jedoch nicht gehemmt.

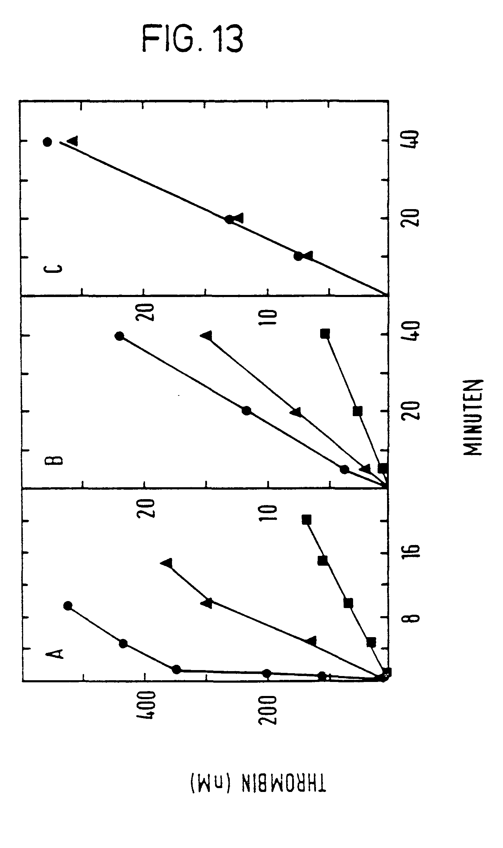

Auf Grund dieser Erfahrungen wurde der Effekt des Antigerinnungsmittels auf die Umsetzung von Prothrombin in Thrombin durch Faktor $X_a$, Faktor $V_a$, Phospholipid und $Ca^{2+}$ untersucht. Unter den genannten experimentellen Bedingungen wird die Thrombinbildung auf eine dosis abhängige Weise durch das Antigerinnungsmittel (siehe Fig. 13A) gehemmt. Die Aktivierung des Prothrombins durch Faktor $X_a$, Phospholipid und $Ca^{2+}$ in Abwesenheit des Faktors $V_a$ kann gleichfalls durch das Antigerinnungsmittel gehemmt werden (siehe Fig. 13B). Diese Inhibition wird jedoch nicht beobachtet, wenn die Aktivierung in Abwesenheit von Phospholipid erfolgt (siehe Fig. 13C).

Beispiel V

Polyklonale Antikörper gegen VAC

Polyklonale Antikörper gegen Rinder VAC wurden in Kaninchen stimuliert. Rinder VAC, nach der oben beschriebenen Methode gereinigt, wurde mit gleichen Anteilen an komplettem Freud'schem Adjuvanz gemischt. Die Mischung wurde dem Kaninchen subcutan injiziert. Nach 4 Wochen erfolgte eine Auffrischung, indem dem Kaninchen erneut gereinigtes Rinder VAC s.c. injiziert wurde. Diese Booster-Injektion wurde zweimal im Abstand von 2 Wochen wiederholt. 10 Tage nach der letzten Booster-Injektion wurde dem Kaninchen Blut entnommen und das Blut zur Gerinnung gebracht um Serum zu erhalten.

Immunoglobuline (Ig) wurden aus dem Serum nach dem folgenden Schema isoliert.

a) Das Serum wurde 30 Minuten auf 56 °C erhitzt.

b) Anschließend wurde das Serum auf DEAE-Sephacel aufgetragen, das mit 50 mM TRIS, 100 mM NaCl, pH 8,2 äquilibriert worden war.

c) Das nicht-gebundene Protein wird mit $(NH_4)_2SO_4$ bei 50 %iger Sättigung mit $(NH_4)_2SO_4$ präzipitiert.

d) Die präzipitierten Protein wurden durch Zentrifugieren pelletiert, das Pellet wird resuspendiert in 50 mM Tris, 100 mM NaCl pH 7.9 und ausgiebig gegen den gleichen Puffer dialysiert.

e) Die erhaltene Proteinmischung enthält das Ig.

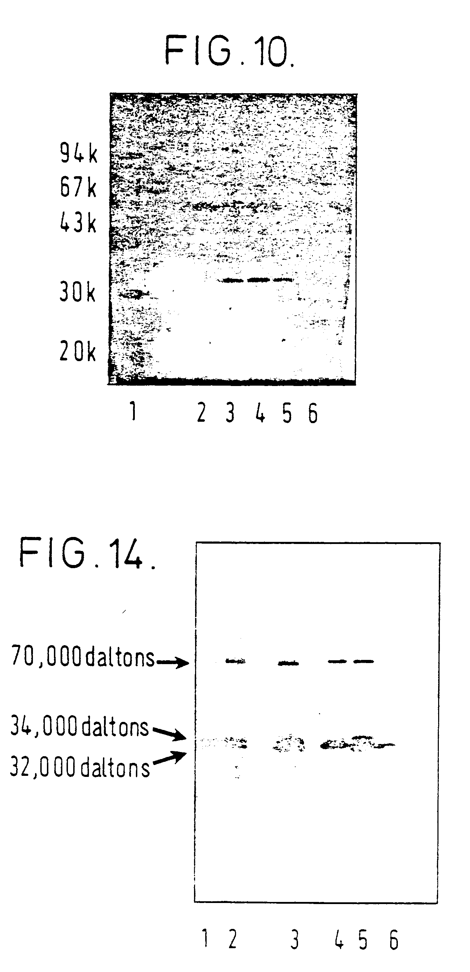

Aus Rinder-Aorta, Rinder-Lunge, Ratten- und Pferde-Aorta und menschlichen Nabelschnurarterien wurden Proteinfraktionen nach den beschriebenen Verfahren isoliert, die VAC-Aktivität zeigen. Die Proteine wurden auf einem Polyacrylamidgel in Gegenwart von Dodecylsulfat und unter nichtreduzierenden Bedingungen durch Elektrophorese getrennt. Nach Beendigung der Elektrophorese wurden die Proteine von dem Gel auf Nitrozellulose Streifen, wie bei Towbin et al. (21) beschrieben, übertragen. Die Streifen wurden mit dem Anti-VAC-Ig inkubiert und nach intensivem Waschen mit Ziegen, Anti-Kaninchen-Ig, an dem Meerrettichperoxidase gekuppelt ist, inkubiert. Diese wird mit Diamin-benzidintetrahydrochlorid, ein Substrat für die Peroxidase, sichtbar gemacht. Nach Beendigung des beschriebenen Verfahrens zeigte ein braunes Band auf dem Nitrozellulosestreifen die Gegenwart eines Ziegen-Kaninchen Ig's an. Außerdem waren Anti-VAC-Ig und Proteine, an die die Anti-VAC-Ig binden, auf diesem Fleck vorhanden. Immunoblots der Proteine mit VAC-Aktivität, isoliert aus Rinder-Aorta, Rinder-Lunge, Ratten- und Pferde-Aorta und menschlichen Nabelschnurarterien sind in Figur 14 dargestellt.

Diese Ergebnisse zeigen folgendes : Bei Anwendung des beschriebenen Isolierungsschemas kann eine Protein-Fraktion mit VAC-Aktivität aus Rinder-Aorten, Rinder-Lungen, Ratten- und Pferde-Aorten und menschlichen Nabelschnurarterien erhalten werden. Weiterhin : die isolierten Proteinfraktionen mit Vac-Aktivität enthalten Proteine mit Molekulargewichten von ± 32 000, ± 34 000 und ± 70 000, die mit Anti-VAC-Ig, das gegen gereinigtes Rinder VAC in Kaninchen stimuliert worden war, reagieren.

Beispiel VI

Reinigungsschritt für VAC unter Verwendung großvolumiger Phospholipid Vesikel

Großvolumige Phospholipid-Vesikel (LVV), zusammengesetzt aus 1,2-Dioleoyl-sn-glycero-3-phosphoserin (PS) und 1,2-Dioleoyl-sn-glycero-3-phosphocholin (PC) wurden wie bei P. van de Waart et al. (20) beschrieben, hergestellt. Für den Reinigungsschritt wurden LVV, enthaltend PS/PC (Molverhältnis 20 : 80) verwendet. Doch sind auch andere Molverhältnisse zu verwenden, mit der Einschränkung, daß negativ geladene Phospholipide vorhanden sein müssen. Ebenso kann die Kettenlänge der Fettsäuren in den Phospholipiden variiert werden.

Verfahren

LVV, ± 1 mM Phospholipid in 50 mM Tris/HCl, 100 mM NaCl, pH 7,9, wurden mit einem gleichen Volumen einer Proteinfraktion, die VAC-Aktivität enthält, vermischt. Die Proteine sind in 50 mM Tris/HCl, 100 mM NaCl, 10 mM $CaCl_2$, pH 7,9 gelöst. Die Mischung wird 5 Minuten bei Raumtemperatur gehalten. Anschließend wird die Mischung 30 Minuten bei 20 000xg zentrifugiert. Das Pellet wird resuspendiert in 50 mM Tris/HCl, 100 mM NaCl, 10 mM $CaCl_2$, pH 7,9 und erneut zentrifugiert. Das erhaltene Pellet wird anschließend in 50 mM Tris/HCl, 100 mM NaCl, 10 mM Ethylendiamintetraessigsäure (EDTA), pH 7,9, resuspendiert und wieder zentrifugiert. Der erhaltene Überstand enthält die VAC-Aktivität. Die oben beschriebene Methode ist ein effizienter Reinigungsschritt bei dem Verfahren, gereinigtes VAC zu erhalten.

Figurbeschreibung

Fig. 1 Gelfiltration von VAC auf Sephadex G-100.

Die Säule (3 × 80 cm) wurde mit einer Druckhöhe von 60 cm hergestellt und mit 500 mM NaCl und 20 mM Tris/HCl, pH 7,5 äquilibriert. Die VAC enthaltende Fraktion, erhalten nach der DEAE-Chromatographie wurde bis 2 ml konzentriert und danach auf das Sephadex G-100 geführt. Die Druckhöhe von 60 cm wurde aufrechterhalten. Das leere Volumen war 245 ml (Fraktion 70). Hiernach wurden die Fraktionen von 2 ml gesammelt. Die Fraktionen wurden gegen 10 % Glycerol enthaltendes TBS dialysiert wonach sie, wie in Beispiel I beschrieben nach dem Einstufenkoagulationsversuch auf VAC-Aktivität geprüft wurden. Die Gerinnungszeiten wurden bei Verdünnungen von 1 : 10 der G-100 Fraktionen mit TBS erhalten. Die Gerinnungszeit in Abwesenheit von VAC war 65 Sekunden.

Fig. 2. Analytische SDS-PAGE von VAC.

SDS-PAGE mit Gelen, welche 10 Gew.% Acrylamid, 0,27 Gew.% N,N[3]-Methylenbisacrylamid und 0,1 % SDS enthalten, wurde nach Laemmli ausgeführt (Laemmli, U.K. (1970) Nature 227, 680-685). Die Bedeutung der Zahlen auf der x-Achse ist wie folgt :
1) Materialien mit Normmolekulargewicht, bei denen die Disulfidbindungen reduziert sind,
2) 25 $\mu$g reduzierter VAC,
3) 25 $\mu$g nicht-reduzierter VAC.
Das Gel wurde gefärbt mit Coomassie Blue und auf die in Beispiel I beschriebene Weise entfärbt

Fig. 3. Die Bestimmung des isoelektrischen pH von VAC.

Die Elektrofokussierung wurde mit PAGE-Platten in dem pH-Bereich von 3,5-9,5 (vergleiche Beispiel I) ausgeführt. 200 $\mu$g menschliches $H_b^1$ und 20 $\mu$g VAC wurden auf das Gel gebracht, nachdem der pH-Gradient in dem Gel gebildet war. Menschliches $H_b$ diente als Referenz mit dem bekannten isoelektrischen Punkt bei pH 6,8. Bevor das Gel der Färbung mit Coomassie Blue ausgesetzt wurde, wurde das Gel während 30 Minuten mit 0,7 M Trichloressigsäure fixiert.

Fig. 4. Die Analyse der Bindung von VAC an negativ geladenen Phospholipid-Liposomen mit SDS-PAGE

SDS-PAGE wurde nach Laemmli ausgeführt (Laemmli, U.K. (1970) Nature 227, 680-685) auf den gleichen Plättchen wie bei der Figur 2 beschrieben. Die analysierten Proben wurden aus den Bindungsexperimenten erhalten, wie in der Erläuterung bei Tabelle B erwähnt. Die Bedeutung der Zahlen auf der x-Achse ist wie folgt :

1) Materialien mit Norm-Molekulargewicht, bei denen die Disulfidbindungen reduziert sind,

2) obenstehende Flüssigkeit, erhalten nach dem Zentrifugieren eines VAC-Präparates in Abwesenheit von Liposomen,

3) obenstehende Flüssigkeit, erhalten nach dem Zentrifugieren von VAC in Gegenwart von Liposomen,

4) obenstehende Flüssigkeit, erhalten nach dem Zentrifugieren von VAC in Gegenwart von Liposomen und $Ca^{++}$ und

5) obenstehende Flüssigkeit, erhalten nach dem Zentrifugieren des Liposom-Präzipitats von 4), das aufs neue suspendiert wurde in 10 mM EDTA enthaltendem TBS.

Fig. 5. Der Effekt der Konzentration von VAC auf die Inhibition (%) der Thrombin-Bildung

Die erwähnten Konzentrationen von VAC sind die endgültig in den Versuchssystemen vorhandenen Konzentrationen. Die Thrombin-Bildung wurde gemessen bei 1 $\mu$M Prothrombine, 10 nM Faktor $X_a$ und 0,5 M (▲-▲) oder 5 M (●-●) Phospholopid-Membran (PC/PS ; 4 : 1, Mol/Mol) in 10 mM $CaCl_2$ enthaltendem TBSA. Das Reaktionsgemisch wurde mit den erwähnten Mengen VAC (S.A. 1 300 Einheiten/mg) während 3 Minuten bei 37 °C ohne Prothrombin gerührt. Durch die Hinzufügung von Prothrombin zu dem Gemisch wurde wie in Beispiel I die Thrombin-Bildung ausgelöst, bei der die Geschwindigkeit gemessen wurde. Die Geschwindigkeit der Thrombin-Bildung in Abwesenheit von VAC betrug 3,3 nM $II_a$/Min. (▲-▲) beziehungsweise 10,9 nM $II_a$/Min. (●-●).

Fig. 6. Der Effekt der Phospholipidkonzentration auf die Inhibition (%) der Thrombin-Bildung durch VAC.

Die Thrombin-Bildung wurde gemessen bei 1 $\mu$M Prothrombin, 10 nM Faktor $X_a$, 10,7 $\mu$g/ml VAC (S.A. 1 300 Einheiten/mg) und verschiedenen Konzentrationen Phospholipid-Membran (PC/PS ; 4 : 1, Mol/Mol) in TBSA. Faktor $X_a$, VAC und Phospholipid wurden 3 Minuten bei 37 °C in TBSA gerührt. Die Thrombin-Bildung wurde ausgelöst durch die Hinzufügung von Prothrombin zu dem Reaktionsgemisch. Die Geschwindigkeit der Thrombin-Bildung wurde gemessen, wie in Beispiel I beschrieben. Der Prozentsatz der Inhibition der Thrombin-Bildung (●-●) wurde für jede Phospholipidkonzentration mit der entsprechenden Geschwindigkeit der Thrombin-Bildung bei Abwesenheit von VAC (▲-▲) gemessen.

Fig. 7. Gel-Filtration eines 10.000 xg Überstandes von einem Nabelschnurarterien-Homogenat auf Sephadex G-100

2 ml eines 10.000 xg Überstandes wurden auf eine Sephadex G-100 Säule (1.5 × 80 cm), die mit TBS pre-äquilibriert war, aufgetragen. Die Säule wurde mit TBS eluiert. Aliquote der erhaltenen Fraktionen wurden im MPTT getestet. Bestimmte Fraktionen (□) zeigen eine Prokoagulant-Aktivität und lösten die Gerinnung im MPTT aus, ohne Zugabe von HTP, Factor $X_a$ oder Thrombin. Andere Fraktionen (□) verlängern die Gerinnungszeit im MPTT bei Verwendung von HTP als Auslöser der Gerinnung. Diese Fraktionen wurden gesammelt und weiterfraktioniert.

Fig. 8. Chromatographie des Antikoagulanten auf DEAE-Sephacel (A) und Sephadex G-75 (B)

Der Pool von der Sephadex G-100 Säule, das Antikoagulant enthaltend, wurde auf DEAE-Sephacel gebracht. Elution wurde mit 200 ml eines linearen Gradienten von 50 mM-300 mM NaCl (---) vorgenommen. Fraktionen (4 ml) wurden gesammelt und auf $A_{280}$ (—) und Antikoagulant-Aktivität im MPTT unter Verwendung von HTP (Endkonzentration 95 $\mu$g Protein/ml) als Starter für die Gerinnung (●) überprüft. Die Fraktionen mit Antikoagulant-Aktivität wurden gesammelt, konzentriert und anschließend auf Sephadex G-75 (B) gebracht. 2 ml Fraktionen wurden gesammelt und auf $A_{280}$ (—) und Antikoagulant-Aktivität (●) überprüft. $V_0$ kennzeichnet das Leervolumen der Säule.

16

Fig. 9. Dosis-Abhängigket des Antikoagulanten im MPTT

Variierende Mengen an Antikoagulant wurden dem MPTT zugesetzt. Koagulation wurde durch HTP ausgelöst (Endkonzentration 95 $\mu$g Protein/ml). Die Kontroll-Gerinnungszeit war 65 Sekunden.

Fig. 10. Gel-Elektrophorese verschiedener Fraktionen des G-75-Eluates

Eine bestimmte Menge an verschiedenen Fraktionen des G-75-Eluates wurde, wie beschrieben, einer Gel-Elektrophorese unterzogen. Die Gele wurden wie bei Merril et al. (12) beschrieben, mit Silber angefärbt (12). Spalte 1 : reduzierte niedrig-molekulare Standards ; Spalten 2-6 : unreduzierte Aliquote von G-75-Fraktionen mit den Nummem 35, 39, 41, 43 und 50.

Fig. 11. Effekt proteolytischer Enzyme auf die Aktivität des Antikoagulanten

Das Antikoagulant wurde bei 37 °C mit Protease Typ I (■, Endkonzentration 0,11 Einheiten/ml), Trypsin (▲, Endkonzentration 88 BAEE Einheiten/ml) und ohne proteolytische Enzyme (●) inkubiert. Zu angegebenen Zeiten wurden 5 $\mu$l, 6 $\mu$g Antikoagulant-Protein enthaltend, aus der Reaktionsmischung entnommen und dem MPTT zugegeben. Die Gerinnung wurde mit HTP ausgelöst (Endkonzentration 18 Protein $\mu$g/ml). Kontrollgerinnungszeit betrug 110 sec. Die angegebenen Einheiten für die proteolytischen Enzyme sind aus Angaben der Hersteller abgeleitet.

Fig. 12. Effekt des Vascular Antikoagulant auf die Gerinnungszeit die im M(P)TT entweder durch HTP, Faktor $X_a$ oder Thrombin induziert wurde

Die Konzentrationen der Gerinnungsstarter (HTP 18 $\mu$g Protein/ml 1,5 nM Faktor $X_a$ oder 0,4 nM Thrombin) wurden so gewählt, daß die Kontrollgerinnungszeit etwa 110 sec. betrug (offene Balken). Bei Verwendung von Faktor $X_a$ wurden Phospholipid Vesikel (Endkonzentration 10 $\mu$M) zusammengesetzt aus $Ole_2$Gro-P-Ser/$Ole_2$Gro-P-Cho (Molverhältnis 20 : 80) der Reaktionsmischung zugesetzt. Gerinnungszeiten, die durch die angegebenen Agentien in Gegenwart von 3,5 $\mu$g Antikoagulant-Protein ermittelt wurden, sind als schattierte Balken dargestellt.

Fig. 13. Effekt des Antikoagulanten auf die Prothrombin Aktivierung durch ($X_a$, $V_a$, Phospholipid, $Ca^{2+}$), ($X_a$, Phospholipid, $Ca^{2+}$) und ($X_a$, $Ca^{2+}$)

Die Reaktionsmischungen enthielten : (A) 1 $\mu$M Prothrombin, 0,3 nM $X_a$, 0.6 nM $V_a$, 0,5 $\mu$M Phospholipid und 10 mM $CaCl_2$ mit 12,0 $\mu$g/ml Antikoagulant (■), 4,8 $\mu$g/ml Antikoagulant (▲) und kein Antikoagulant (●). (B) 1 $\mu$M Prothrombin, 10 nM $X_a$, 0.5 $\mu$M Phospholipid und 10 nM $CaCl_2$ mit 2,4 $\mu$g/ml Antikoagulant (■), 0,48 $\mu$g/ml Antikoagulant (▲) und kein Antikoagulant (●). (C) 1 $\mu$M Prothrombin, 75 nM $X_a$ and 10 mM $CaCl_2$ mit 120 $\mu$g/ml Antikoagulant (▲) und kein Antikoagulant (●). Zu den angegebenen Zeiten wurden Proben entnommen und das Thrombin wie beschrieben bestimmt.

Fig. 14. Immunoblots

Die Blots wurden wie oben beschrieben erhalten.

Spalte 1 :     Protein Fraktion mit VAC-Aktivität, isoliert aus Rinder-Aorta.
Spalte 2 :     Protein Fraktion mit VAC-Aktivität, isoliert aus Rinder-Aorta.
Spalte 3 :     Protein Fraktion mit VAC-Aktivität, isoliert aus Rinder-Lunge.
Spalte 4 :     Protein Fraktion mit VAC-Aktivität, isoliert aus menschlichen Nabelschnurarterien.
Spalte 5 :     Protein Fraktion mit VAC-Aktivität, isoliert aus Ratten-Aorta.
Spalte 6 :     Protein Fraktion mit VAC-Aktivität, isoliert aus Pferde-Aorta.

Fig. 15. Die Gelelektrophorese (A) und die Antigerinnungsaktivität (B) der verschiedenen Fraktionen des Eluats aus G-75.

Bestimmte Mengen der verschiedenen Fraktionen des Eluats aus G-75 wurden der Gel-Elektrophorese ausgesetzt, wie vorstehend beschrieben worden ist. Die Banden wurden durch Silber nach der Methode von Merril C. R. Goldman D., & Van Keuren M. L. (1982) Electrophoresis 3, 17-23 angefärbt. Elektrophorese-Bahn 1 : Normmaterial mit einem niedrigen Molekulargewicht ; Elektrophorese-Bahnen 2-6 : nicht-reduzierte

EP 0 181 465 B2

gleiche Mengen der G-75 Fraktionen, bei wachsenden Elutionsvolumina. Bestimmte Mengen der G-75 Fraktionen, die mit der Gel-Elektrophorese analysiert worden waren, wurden in dem MPTT geprüft (siehe vorstehende Beschreibung) unter Anwendung von HTP zur Auslösung der Gerinnung. Die Kontrollgerinnungszeit ist durch den offenen Balken dargestellt. Die Zahlen unter den schraffierten Balken entsprechen den Zahlen der Elektrophoresebahnen in Fig. 15A.

Fig. 16. Die Hitze-Inaktivierung des vaskulären Antigerinnungsmittels

Das Antigerinnungsmittel wurde bei 56 °C inkubiert und nach den verschiedenen Inkubationszeiten wurde je eine Probe von 5 $\mu$l, welche 3,6 $\mu$g Protein enthielt, entnommen, sofort mit Eis abgekühlt und danach in dem MPTT unter Verwendung von HTP als Koagulationsauslöser geprüft. Die Gerinnungszeit der Kontrollprobe war 110 Sekunden.

Literatur

1. Jackson, C. M. & Nemerson, Y (1980) Ann. Rev. Biochem. 49, 765-811
2. Rosing, J., Tans, G., Govers-Riemslag, J. W. P., Zwaal, R.F.A. & Hemker, H. C. (1980) J. Biol. Chem. 255, 274-283
3. Dieijen, G. van, Tans, G., Rosing, J. & Hemker, H. C. (1981) J. Biol. Chem. 256, 3433-3442
4. Rosenberg, R. D. & Rosenberg, J. S. (1984) J. Clin Invest. 74, 1-6
5. Kurachi, K., Fujikawa, K., Schmer, G. & Davie, E. W. (1976) Biochemistry 15, 373-377
6. Ellis, V., Scully, M., Macgregor, I. & Kakkar, V. (1982) Biochim. Biophys. Acta 701, 24-31
7. Stenflo, J. (1976) J. Biol. Chem. 251, 355-363
8. Walker, F. J. Sexton, P. W. & Esmon, C. T. (1979) Biochim. Biophys. Acta 571, 333-342
9. Vehar, G. A. & Davie, E. W. (1980) Biochemistry 19, 401-409.
10. Nossel, H. L. (1981) Nature (Lond.) 291, 165-167
11. Laemmli, U. K. (1970) Nature 227, 680-685
12. Merril, C. R. Goldman, D. & Keuren, M. L. van (1982) Electrophoresis 3, 17-23
13. Hornstra, G. (1981) Phil. Trans. R. Soc. Lond B 294, 355-371
14. Dam-Mieras, M. C. E. van, Muller, A. D., Dieijen, G. van & Hemker, H. C. (1984) Methods of Enzymatic Analysis, vol. 5, pp. 352-365, Verlag Chemie GmbH, Weinheim, F.R.G.
15. Lindhout, T., Govers-Riemslag, J. G., Waart, P. van de, Hemker, H. C. & Rosing, F. (1982) Biochemistry 21, 4594-5502
16. Owen, W. G., Esmon, C. T. & Jackson, C. M. (1974) J. Biol. Chem. 249, 594-605
17. Nesheim, E. M., Myrmel, K. H., Hibbard, L. & Mann, K. G. (1979) J. Biol. Chem. 254, 508-517
18. Lowry, O. H., Rosebrough, N. V., Farr, A. L & Randall, R. J. (1951) J. Biol. Chem. 193, 265
19. Böttcher, C. J. F., Gent, C. M. van & Pries, C. (1961) Anal. Chim. Acta 24, 203-207
20. Waart, P. van de ; Bruls, H., Hemker, H. C. ; Lindhout, Th. ; Biochemistry (1983), 22, 2427-2432
21. Towbin, H., Staehelin, Th., Gordon, J. : Proc. Natl. Acad. Sci. USA, 76, 4350-4354 (1979).

**Patentansprüche**

1. Blutgerinnungshemmende Proteine, dadurch gekennzeichnet, daß sie
   - keine Glykoproteine sind
   - aus stark vaskularisiertem Gewebe isolierbar sind,
   - die Gerinnungsfaktoren nicht inaktivieren,
   - Phospholipide nicht hydrolysieren,
   - über die divalenten Kationen $Ca^{2+}$ und/oder $Mn^{2+}$ reversibel an negativ geladene Phospholipide binden,
   - in der Lage sind, den Faktor $X_a$ und die Prothrombine von einer negativ geladenen Phospholipidoberfläche zu verdrängen und
   - nicht die biologische und amidolytische Aktivität der Faktoren $X_a$ und $II_a$ hemmen.

2. Blutgerinnungshemmende Proteine nach Anspruch 1, dadurch gekennzeichnet, daß sie die durch ein vaskulares Prokoagulanz bzw. durch den Faktor $X_a$ induzierte Gerinnung hemmen, jedoch nicht die von Thrombin induzierte Gerinnung.

3. Blutgerinnungshemmende Proteine nach Anspruch 1, dadurch gekennzeichnet, daß sie

EP 0 181 465 B2

- den modifizierten Prothrombin-Zeit-Versuch und/oder
- den modifizierten, aktivierten, partiellen Thromboplastin-Zeit-Versuch und/oder
- den nicht-modifizierten Prothrombin-Zeitversuch und/oder
- die Prothrombin-Aktivierung durch den Gerinnungsfaktor $X_a$ in Gegenwart von negativ geladenen Phospholipiden und $Ca^{2+}$ und/oder
- die intrinsische X-Aktivierung durch den Faktor $IX_a$ in Gegenwart von negativ geladenen Phospholipiden und $Ca^{2+}$ und/oder
- die Prothrombin-Aktivierung von isolierten, stimulierten Blutplättchen und/oder
- die von der Gefäßwand induzierte Gerinnung und/oder
- die gerinnungsabhängige Plättchenaggregation hemmen.

4. Blutgerinnungshemmende Proteine nach Anspruch 1, dadurch gekennzeichnet, daß
   - deren hemmende Wirkung von der Menge an Phospholipiden abhängt und
   - die durch die Proteine induzierte Prothrombin-Aktivierungshemmung durch den Faktor $X_a$ abhängig ist von der Phospholipidkonzentration.

5. Blutgerinnungshemmende Proteine nach Anspruch 1, dadurch gekennzeichnet, daß sie Molekulargewichte von ca. $70 \times 10^3$, ca. $60 \times 10^3$, ca. $34 \times 10^3$ oder ca. $32 \times 10^3$ aufweisen.

6. Blutgerinnungshemmende Proteine nach Anspruch 1, dadurch gekennzeichnet, daß sie aus Arterien isolierbar sind.

7. Blutgerinnungshemmende Proteine nach Anspruch 1, dadurch gekennzeichnet, daß
   - sie aus Blutgefäßwänden von Säugetieren isoliert und gereinigt wurden,
   - sie keine Glykoproteine sind,
   - sie keine Phospholipasen sind,
   - sie einen isoelektrischen Punkt bei pH 4,4-4,6 besitzen,
   - die Aktivität der blutgerinnungshemmenden Protein bei 56 °C thermolabil ist,
   - die Aktivität der blutgerinnungshemmenden Proteine im zitrierten Plasma bei 37 °C einige Stunden stabil bleibt,
   - die Aktivität der blutgerinnungshemmenden Proteine von Trypsin und/oder Chymotrypsin nicht vollständig zerstört wird.
   - die Aktivität der blutgerinnungshemmenden Proteine von Collagenase und/oder Elastase nicht beeinflußt wird.
   - sie über die divalenten Kationen $Ca^{2+}$ und/oder $Mn^{2+}$ mit negativ geladenen Phospholipiden, die in Vesikeln, Liposomen oder Etherosomen zu finden sind, binden,
   - sie über die divalenten Kationen $Ca^{2+}$ und/oder $Mn^{2+}$ mit negativ geladenen Phospholipiden, die mit Spherocil gekoppelt sind, binden,
   - die Bindung der Proteine an die negativ geladenen Phospholipide reversibel ist und von Ethylendiamintetraessigsäure (EDTA) aufgehoben werden kann.
   - sie den modifizierten Prothrombin-Zeit-Versuch hemmen,
   - sie den modifizierten, aktivierten, partiellen Thromboplastin-Zeit-Versuch hemmen,
   - sie den nicht-modifizierten Prothrombin-Zeifversuch hemmen,
   - sie die Prothrombin-Aktivierung durch den Gerinnungsfaktor $X_a$ in Gegenwart von negativ geladenen Phospholipiden und $Ca^{2+}$ in vitro hemmen,
   - sie die intrinsische X-Aktivierung durch den Faktor $IX_a$ in Gegenwart von negativ geladenen Phospholipiden und $Ca^{2+}$ in vitro hemmen,
   - sie die Prothrombin-Aktivierung von isolierten, stimulierten Blutplättchen in vitro hemmen,
   - sie die von der Gefäßwand induzierte Gerinnung in vitro hemmen und
   - die durch die Proteine induzierte Prothrombin-Activierungshemmung durch den Faktor $X_a$ von der Phospholipidkonzentration abhängig ist und bei hohen Phospholipidkonzentrationen kleiner ist.

8. Blutgerinnungshemmende Proteine nach einem oder mehreren der vorhergehenden Ansprüche.

9. Humane blutgerinnungshemmende Proteine nach einem der vorhergehenden Ansprüche.

10. Bovine blutgerinnungshemmende Proteine nach einem der vorhergehenden Ansprüche.

19

**11.** Murine blutgerinnungshemmende Proteine nach einem der vorhergehenden Ansprüche.

**12.** Equine blutgerinnungshemmende Proteine nach einem der vorhergehenden Ansprüche.

**13.** Verfahren zur Herstellung blutgerinnungshemmender Proteine gemäß einem der Ansprüche 1 bis 8, dadurch gekennzeichnet, daß Blutgefäßwände, stark vaskularisierte Gewebe oder Endothelzellkulturen

a) homogenisiert und differentiell zentrifugiert werden und die obenstehende Flüssigkeit in beliebiger Reihenfolge einer oder mehrerer der folgenden Reinigungsbehandlungen ausgesetzt wird:

b) Präzipitieren mit Salz

c) Affinitätschromatographie

d) Ionenaustauschchromatographie

e) Chromatographie über ein Molekularsieb, wobei die nach den Stufen b), c), d) und e) erhaltenen Produkte gewünschtenfalls dialysiert werden können.

**14.** Verfahren nach Anspruch 13, dadurch gekennzeichnet, daß eine weitere Reinigung mit Hilfe einer Immunoadsorptionschromatographie durchgeführt wird.

**15.** Verfahren nach Anspruch 13 oder 14, dadurch gekennzeichnet, daß die Proteine mit Hilfe von Phospholipid-Vesikeln gereinigt werden.

**16.** Verfahren nach einem der Ansprüche 13 bis 15, dadurch gekennzeichnet, daß für die Präzipitation in der Stufe b) Ammoniumsulfat, für die Chromatographie in der Stufe c) Hydroxyapatit, für die Chromatographie in der Stufe d) DEAE Sephacel und für die Chromatographie in der Stufe e) Sephadex G-100 bzw. G-75 verwendet wird.

**17.** Blutgerinnungshemmendes Protein, herstellbar nach einem der Ansprüche 13 bis 16.

**18.** Pharmazeutisches Präparat, dadurch gekennzeichnet, daß es neben pharmazeutisch inerten Hilfs- und/oder Trägerstoffen eine wirksame Menge an blutgerinnungshemmendem Protein nach einem der Ansprüche 1 bis 12 und 17 enthält.

**19.** Verwendung des blutgerinnungshemmenden Proteins nach einem der Ansprüche 1 bis 12 und 17 zur Herstellung eines antithrombotischen Arzneimittels.

**Claims**

**1.** Blood coagulation-inhibiting proteins, characterised in that
- they are not glyco-proteins,
- they are capable of being isolated from strogly vascularised tissue,
- they do not deactivate the coagulation factors,
- they do not hydrolyse phospholipids,
- they bind reversibly to negatively charged phospholipids via the divalent cations $Ca^{2+}$ and/or $Mn^{2+}$,
- they are capable of displacing the factor $X_a$ and the prothrombins from a negatively charged phospholipid surface and
- they do not inhibit the biological and amidolytic activity of the factors $X_a$ and $II_a$.

**2.** Coagulation-inhibiting proteins according to claim 1, characterised in that they inhibit the coagulation induced by a vascular procoagulant or by the factor $X_a$ but they do not inhibit thrombin-induced coagulation.

**3.** Coagulation-inhibiting proteins according to claim 1, characterised in that they inhibit
- the modified prothrombin-time experiment and/or
- the modified, activated, partial thromboplastin-time experiment and/or
- the non-modified prothrombin-time experiment and/or
- the prothrombin activation by the coagulation factor $X_a$ in the presence of negatively charged phospholipids and $Ca^{2+}$ and/or

EP 0 181 465 B2

- the intrinsic X-activation by the factor $IX_a$ in the presence of negatively charged phospholipids and $Ca^{2+}$ and/or
- the prothrombin activation of isolated, stimulated blood platelets and/or
- the coagulation induced by the vessel wall and/or
- the coagulation-dependent platelet aggregation.

4. Coagulation-inhibiting proteins according to claim 1, characterised in that
- their inhibitory effect depends on the quantity of phospholipids and
- the protein-induced inhibition of prothrombin activation by the factor $X_a$ is dependent on the phospholipid concentration.

5. Coagulation-inhibiting proteins according to claim 1, characterised in that they have molecular weights of about $70 \times 10^3$, about $60 \times 10^3$, about $34 \times 10^3$ or about $32 \times 10^3$.

6. Coagulation-inhibiting proteins according to claim 1, characterised in that they can be isolated from arteries.

7. Coagulation-inhibiting protein according to claim 1, characterised in that
- they are isolated from the walls of the blood vessels in mammals and purified,
- they are not glycoproteins,
- they are not phospholipases,
- they have an isoelectric point at pH 4.4 to 4.6,
- the activity of the coagulation-inhibiting proteins at 56 °C is thermally unstable,
- the activity of the coagulation-inhibiting proteins in citrated plasma remains stable for some hours at 37 °C,
- the activity of the coagulation-inhibiting proteins is not completely destroyed by trypsin and/or chymotrypsin,
- the activity of the coagulation-inhibiting proteins is not affected by collagenase and/or elastase,
- they bond, via the divalent cations $Ca^{2+}$ and/or $Mn^{2+}$, to negatively charged phospholipids which can be found in vesicles, liposomes or etherosomes,
- they bond, via the divalent cations $Ca^{2+}$ and/or $Mn^{2+}$, to negatively charged phospholipids which are coupled to Spherocil,
- the bonding of the proteins to the negatively charged phospholipids is reversible and can be removed by ethylenediamine tetraacetic acid (EDTA),
- they inhibit the modified prothrombin-time experiment,
- they inhibit the modified, activated, partial thromboplastin-time experiment, - they inhibit the non-modified prothrombin-time experiment,
- they inhibit the activation of prothrombin by the coagulation factor $X_a$ in the presence of negatively charged phospholipids and $Ca^{2+}$ in vitro,
- they inhibit the intrinsic X-activation by the factor $IX_a$ in the presence of negatively charged phospholipids and $Ca^{2+}$ in vitro,
- they inhibit the prothrombin activation of isolated stimulated blood platelets in vitro,
- they inhibit the coagulation induced by the blood vessel wall in vitro and
- the protein-induced inhibition of prothrombin activation by factor $X_a$ is dependent on the phospholipid concentration and is smaller at high phospholipid concentrations.

8. Coagulation-inhibiting proteins according to one or more of the preceding claims.

9. Human coagulation-inhibiting proteins according to one of the preceding claims.

10. Bovine coagulation-inhibiting proteins according to one of the preceding claims.

11. Murine coagulation-inhibiting proteins according to one of the preceding claims.

12. Equine coagulation-inhibiting proteins according to one of the preceding claims.

13. Process for preparing a coagulation-inhibiting protein according to one of claims 1 to 8, characterised in that blood vessel walls, strongly vascularised tissue or endothelial cell cultures

21

a) are homogenised and differentially centrifuged and the supernatant liquid is subjected to one or more of the following purification treatments in any desired sequence:
b) precipitation with salt
c) affinity chromatography
d) ion exchange chromatography
e) chromatography over a molecular sieve, whilst the products obtained according to stages b), c), d) and e) may, if desired, be dialysed.

14. Process as claimed in claim 13, characterised in that further purification is carried out using immunoadsorption chromatography.

15. Process according to claim 13 or 14, characterised in that the proteins are purified using phospholipid vesicles.

16. Process according to one of claims 13 to 15, characterised in that ammonium sulphate is used for the precipitation in stage b), hydroxyapatite is used for the chromatography in stage c), DEAE Sephacel is used for the chromatography in stage d) and Sephadex G-100 or G-75 is used for the chromatography in stage e).

17. Coagulation-inhibiting protein prepared according to one of claims 13 to 16.

18. Pharmaceutical preparation, characterised in that it contains, in addition to pharmaceutically inert carriers and/or excipients, an effective quantity of anti-coagulant protein as claimed in one of claims 1 to 12 and 17.

19. Use of the anti-coagulant protein according to one of claims 1 to 12 and 17 for the preparation of an antithrombotic pharmaceutical composition.

**Revendications**

1. Protéines inhibant la coagulation du sang, caractérisées en ce que
   - elles ne sont pas des glycoprotéines,
   - elles peuvent être isolées à partir de tissus fortment vascularisés,
   - elles n'inactivent pas les facteurs de coagulation,
   - elles n'hydrolysent pas les phospholipides,
   - elles se tient, par l'intermédiaire des cations bivalents $Ca^{2+}$ et/ou $Mn^{2+}$ de façon réversible aux phospholipides chargés négativement,
   - elles sont capables de déplacer le facteur $X_a$ et les prothrombines d'une surface de phospholipides chargée négativement et
   - elles n'inhibent pas l'activité biologique et amidolytique des facteurs $X_a$ et $II_a$.

2. Protéines inhibant la coagulation du sang selon la revendication 1, caractérisées en ce qu'elles inhibent la coagulation induite par un procoagulant vasculaire ou respectivement par le facteur $X_a$, mais non la coagulation induite par la thrombine.

3. Protéines inhibant la coagulation du sang selon la revendication 1, caractérisées en ce qu'elles inhibent :
   - l'essai de temps de prothrombine modifiée et/ou
   - l'essai de temps thromboplastine partielle, activée, modifiée et/ou
   - l'essai de temps de prothrombine non-modifiée et/ou
   - l'activation de la prothrombine par le facteur de coagulation $X_a$ en présence de phospholipides chargés négativement et de $Ca^{2+}$ et/ou
   - l'activation intrinsèque de X par le facteur $IX_a$ en présence de phospholipides chargés négativement et de $Ca^{2+}$ et/ou
   - l'activation de la prothrombine par des plaquettes sanguines stimulées, isolées et/ou
   - la coagulation induite par la paroi des vaisseaux et/ou
   - l'aggrégation plaquettaire dépendante de la coagulation.

22

EP 0 181 465 B2

4. Protéines inhibant la coagulation du sang selon la revendication 1, caractérisées en ce que
   - leur activité inhibitrice dépend de la quantité de phospholipides et
   - l'inhibition par les protéines de l'activation de la prothrombine induite par le facteur $X_a$ est dépendante de la concentration en phospholipides.

5. Protéines inhibant la coagulation du sang selon la revendication 1, caractérisées en ce qu'elles présentent un poids moléculaire d'environ $70 \times 10^3$, environ $60 \times 10^3$, environ $34 \times 10^3$ ou environ $32 \times 10^3$.

6. Protéines inhibant la coagulation du sang selon la revendication 1, caractérisées en ce que l'on peut les isoler à partir des artères.

7. Protéines inhibant la coagulation du sang selon la revendication 1, caractérisées en ce que
   - elles ont été isolées et purifiées à partir de parois de vaisseaux sanguins de mammifères,
   - elles ne sont pas des glycoprotéines,
   - elles ne sont pas des phospholipases,
   - elles possèdent un point isoélectrique à pH 4,4-4,6,
   - l'activité des protéines inhibant la coagulation du sang est thermolabile à 56 °C,
   - l'activité des protéines inhibant la coagulation du sang reste stable quelques heures à 37 °C dans du plasma citraté,
   - l'activité des protéines inhibant la coagulation du sang n'est pas complètement détruite par la trypsine et/ou la chymotrypsine,
   - l'activité des protéines inhibant la coagulation du sang n'est pas influencée par la collagénase et/ou l'élastase,
   - elles se lient, par l'intermédiaire des cations bivalents $Ca^{2+}$ et/ou $Mn^{2+}$ avec des phospholipides chargés négativement, que l'on peut trouver dans des vésicules, des liposomes ou des éthérosomes.
   - elles se lient, par l'intermédiaire des cations bivalents $Ca^{2+}$ et/ou $Mn^{2+}$ avec des phospholipides chargés négativement qui sont couplés avec du Spherocil,
   - la liaison des protéines aux phospholipides chargés négativement est réversible et peut être détruite par l'acide éthylènediaminetétracétique (EDTA),
   - elles inhibent l'essai de temps de prothrombine modifiée,
   - elles inhibent l'essai de temps de thromboplastine partielle, activée, modifiée,
   - elles inhibent l'essai de temps de prothrombine non-modifiée,
   - elles inhibent in vitro l'activation de la prothrombine par le facteur de coagulation $X_a$ en présence de phospholipides chargés négativement et de $Ca^{2+}$,
   - elles inhibent in vitro l'activation intrasèque de X par le facteur $IX_a$ en présence de phospholipides chargés négativement et de $Ca^{2+}$,
   - elles inhibent in vitro l'activation de la prothrombine par des plaquettes sanguines stimulées, isolées,
   - elles inhibent in vitro la coagulation induite par la paroi des vaisseaux, et
   - l'inhibition de l'activation par le facteur $X_a$ de la prothrombine, induite par les protéines est dépendante de la concentration en phospholipides et est plus faible à des concentrations élevées en phospholipides.

8. Protéines inhibant la coagulation du sang selon l'une ou plusieurs des revendications précédentes.

9. Protéines humaines inhibant la coagulation du sang selon l'une des revendications précédentes.

10. Protéines bovines inhibant la coagulation du sang selon l'une des revendications précédentes.

11. Protéines murines inhibant la coagulation du sang selon l'une des revendications précédentes.

12. Protéines d'équidés inhibant la coagulation du sang, selon l'une des revendications précédentes.

13. Procédé pour la préparation de protéines inhibant la coagulation du sang selon l'une des revendications 1 à 8, caractérisé en ce que des parois de vaisseaux sanguins, des tissus fortement vascularisés ou des cultures de cellules endothéliales.

23

a) sont homogénéisés et centrifugés différentiellement et en ce que le liquide surnageant est soumis, dans n'importe quel ordre, à l'un ou plusieurs des traitements de purification suivants:
b) précipitation par du sel,
c) chromatographie d'affinité
d) chromatographie sur résine échangeuse d'ions,
e) chromatographie sur un tamis moléculaire, après les étapes b), c), d) et e), les produits obtenus pouvant éventuellement être dialysés.

14. Procédé selon la revendication 13, caractérisé en ce qu'une purification supplémentaire à l'aide d'une chromatographie d'immunoadsorption est effectuée.

15. Procédé selon la revendication 13 ou 14, caractérisé en ce que les protéines sont purifiées à l'aide de vésicules de phospholipides.

16. Procédé selon l'une des revendications 13 à 15, caractérisé en ce que, pour la précipitation dans l'étape b), on utilise du sulfate d'ammonium, pour la chromatographie dans l'étape c) on utilise de l'hydroxyapatite, pour la chromatographie dans l'étape d) on utilise du DEAE Sephacel et pour la chromatographie dans l'étape e) on utilise du Sephalex G-100 ou du Sephadex G-75.

17. Protéine inhibant la coagulation du sang qui peut être préparée selon l'une des revendications 13 à 16.

18. Préparation pharmaceutique, caractérisée en ce qu'elle contient, à coté d'adjuvants et/ou excipients pharmaceutiquement inertes, une quantité active de protéine inhibant la coagulation du sang selon l'une des revendications 1 à 12 et 17.

19. Utilisation de la protéine inhibant la coagulation du sang selon l'une des revendications 1 à 12 et 17 pour la fabrication d'un médicament anti-thrombotique.

# FIG.1

FIG.2

FIG.3

FIG.4

FIG.5

# FIG. 6

# FIG. 7

# FIG. 8.

# FIG. 9

# FIG. 11

# FIG.12

# FIG. 13

# FIG.10.

94k
67k
43k

30k

20k

1    2 3 4 5 6

# FIG.14.

70,000 daltons →

34,000 daltons →
32,000 daltons →

1 2    3    4 5 6

FIG. 15

Inkubationszeit bei 56°C

FIG.16

36